# EUROPEAN PATENT APPLICATION

(11) **EP 1 319 952 A2**
(43) Date of publication of application: **18.06.2003**
(21) Application number: 02258624.2
(22) Date of filing: 13.12.2002
(51) Int. Cl.: G01N 33/50, A01K 67/027

(54) **Transgenic fatty acid transport (FATP) non-human knockout mammals and uses thereof**

(30) Priority: 13.12.2001 US 341163 P
(71) Applicant: WHITEHEAD INSTITUTE FOR BIOMEDICAL RESEARCH, Cambridge, MA 02142 (US); MILLENIUM PHARMACEUTICALS, INC., Cambridge, MA 02139 (US); Yale University Corporation, New Haven, Connecticut 06511 (US)
(72) Inventor: Lodish, Harvey F., Brookline, MA 02481 (US); Stahl, Andreas, Mountain View, CA 94043 (US); Hirsch, David, Somerset, NY 08873 (US); Gimeno, Ruth E., Wellesley, MA 02481 (US); Shulman, Gerald I., East Haven, CT 06512 (US); Kim, Jason K., Hamden, CT 06518 (US)
(74) Representative: Arends, William Gerrit

(57) **Abstract**

The present invention provides a transgenic Fatty Acid Transport One (FATP1), non-human knockout mammal, *e.g.*, mammal, useful for elucidating the function of FATP in intact animals whose genomes comprise a wild-type FATP gene. Further aspects of the invention provide a method for the identification of agents, *e.g.*, therapeutic agents, that inhibit FATP1 activity, and methods of treating diseases or conditions associated with FATP1 function, *e.g.*, insulin resistance, non-insulin dependent diabetes mellitus, and cellular triglyceride accumulation.

## Description

### BACKGROUND OF THE INVENTION

Non-insulin dependent diabetes (NIDDM) or Type 2 diabetes is the form of diabetes characterized by insulin resistance combined with a relative, rather than an absolute, deficiency of insulin. Insulin resistance can be defined as a diminished ability of insulin to exhibit effective biological activity over a range of concentrations. Individuals suffering from Type 2 diabetes manifest considerable variation in the degree of each of the two defects, from demonstrating predominantly insulin resistance with minimal insulin deficiency to demonstrating predominantly insulin deficiency with minimal insulin resistance.

Insulin resistance is often present as much as ten to twenty years pnor to the actual clinical development of Type 2 diabetes (Shulman, G. 2000, *J*. *Clin. Invest*., 106:171-176). In such cases, high levels of insulin are secreted in an attempt to compensate for the diminished capacity of the insulin to effectively control plasma glucose levels. If the increased secretion is insufficient to provide adequate glucose control, a state of impaired glucose tolerance develops. This state is often followed by an actual decrease in insulin secretion, and the clinical development of Type 2 diabetes.

In addition, studies indicate that the combination of insulin resistance, obesity and elevated plasma levels of free fatty acids are common in both individuals "at nsk" for developing Type 2 diabetes and those with clinical symptoms of the disease (Boden, G., 1999, *Proc. Assoc. Am. Physicians,* 111:241-248). Therefore, it would be extremely useful to understand the role these factors play in the development and pathology of Type 2 diabetes, however, the ability to interpret the relationship of these factors has been hampered by the complexity of their interactions.

### SUMMARY OF THE INVENTION

The present invention is based on the discovery that FATP1 and FATP1-mediated transport of fatty acids plays a pivotal role in insulin resistance in mammals. In particular, it has been determined that FATP1-mediated transport activity, including FATP1 acyl-CoA synthetase activity, can be modulated thereby modulating, *e.g*., insulin sensitivity, cellular fatty acid accumulation, glucose uptake or glucose clearance. Specifically, an agent that inhibits FATP1 activity can be useful in the treatment of non-insulin dependent diabetes mellitus.

The present invention relates to a transgenic non-human mammal, *e.g*., a non-human primate, a sheep, a rat, a mouse, a dog, a cow, a goat, a rabbit or a pig. In particular embodiments, the transgenic non-human mammal is a rodent, *e.g*., a mouse. The genome of the transgenic non-human mammal includes a disruption of a Fatty Acid Transport Protein One (FATP1) gene such that the mammal lacks or has reduced levels of functional FATP1 protein. In a particular embodiment the genome of the transgemc non-human mammal includes a disruption of a FATP1 gene such that the mammal lacks or has reduced levels of functional FATP1 protein, and in which the mammal exhibits an altered insulin/glucose homeostasis and responsiveness to glucose stimulation. The knockout mouse described herein is also referred to herein as a transgenic FATP1 knockout mouse or a FATP1 knockout mouse. The FATP1 knockout mouse of the present invention has at least one non-functional allele for the FATP1 gene. In one embodiment, the FATP1 knockout mouse is characterized by a disruption of the FATP1 gene, which is either a homozygous disruption or a heterozygous disruption.

Encompassed by the present invention are non-human mammals with a genome comprising a disruption of a FATP1 gene such that the mammal lacks or has reduced levels of functional FATP1 protein, and wherein the mammal exhibits an altered insulin/glucose homeostasis and responsiveness to glucose stimulation compared to a wild-type or non-trans genie mammal.

A method of producing the non-human mammal comprises introducing a targeting vector that disrupts the FATP1 gene in an embryonic stem cell, thereby producing a transgenic embryonic stem cell that comprises a disrupted FATP1 gene; selecting a transgenic embryonic stem cell that comprises the disrupted FATP1 gene; introducing the selected transgenic embryonic stem cell into a blastocyst, thereby forming a chimeric blastocyst; and introducing the chimeric blastocyst into the uterus of a pseudopregnant mammal; whereby the pseudopregnant mammal gives birth to a transgenic mammal with a genome comprising a disruption of a FATP1 gene such that the mammal lacks or has reduced levels of functional FATP1 protein, and wherein the resulting mammal exhibits an altered insulin/glucose homeostasis and responsiveness to glucose stimulation compared to a wild-type or non-transgenic mammal.

More specifically, the invention further provides a method of producing a transgenic non-human mammal, *e.g*., a mouse, that lacks a functional FATP1 gene resulting in full or partial loss of FATP1 activity. In this method, a targeting vector is introduced into an embryonic stem cell to produce a transgenic stem cell in which the FATP1 gene is disrupted. A transgenic embryonic stem cell that includes a disrupted FATP1 gene due to the integration of the targeting vector into its genome is then selected. The selected embryonic stem cell is introduced into a blastocyst, thereby forming a chimeric blastocyst, and the chimeric blastocyst is introduced into the uterus of a pseudopregnant mammal wherein the pseudopregnant mammal gives birth to a transgenic mammal with a genome that includes a disruption of a FATP1 gene such that the mammal lacks or has reduced levels of functional FATP1 protein, and wherein the mammal exhibits an altered insulin/glucose homeostasis and responsiveness to glucose stimulation. The method can further comprise breeding the transgenic mammal with a second mammal to generate F1 progeny having a heterozygous disruption of the FATP1 gene, thereby expanding the population of mammals having a heterozygous disruption of the FATP1 gene. The F1 progeny can then be crossbred to produce a transgenic mammal that lacks a functional FATP1 gene due to a homozygous disruption of the FATP1 gene. The present invention further relates to constructs or vectors (*e.g*., FATP1 targeting constructs) designed to disrupt the function of a wild-type murine FATP1 gene. The present invention also provides cells, cell lines, tissues and cellular extracts that lack a functional FATP1 gene.

As a result of the disruption of the FATP1 gene, the knockout mouse of the present invention manifests a particular phenotype. In one embodiment, the FATP1 knockout mouse has altered insulin/glucose homeostasis when compared to a wild-type or non-transgenic mouse. In a particular embodiment, the FATP1 knockout mouse is characterized by increased insulin-stimulated whole body glucose uptake In embodiments described herein, this increase can be exhibited in muscle cells or in liver cells or in both muscle cells and liver cells. In embodiments, the transgenic mouse is characterized by a decrease in fatty acid (*e.g.,* triglyceride) accumulation in muscle cells.

As a result of studies conducted with the FATP1 knockout mice, the invention further relates to the determination that FATP1-mediated transport of fatty acids significantly affects insulin resistance. It has been demonstrated that increased fatty acid delivery to muscle causes muscle-specific insulin resistance. It has also been demonstrated that increased fatty acid delivery to liver causes liver-specific insulin resistance. Applicants have now determined that inhibition of FATP1-mediated transport of fatty acids significantly reduces insulin resistance. Such improvement is demonstrated, for example, by an increase in insulin-stimulated whole body glucose uptake. Such improvement is also demonstrated, for example, by a decrease in triglyceride accumulation in muscle.

The present invention provides screening methods for determining whether an agent inhibits FATP1. The increased insulin sensitivity of the transgenic non-human FATP1 knockout mammals of the instant invention can be utilized as the basis of a screening assay in which an agent is evaluated for its ability to inhibit FATP1 transport of fatty acids into cells. Such transport has been shown herein to increase insulin resistance.

The present invention covers methods for identifying agents that inhibit FATP1 activity comprising the steps of optionally inducing insulin resistance in a non-human mammal or providing an insulin-resistant non-human mammal (*e.g*., a transgenic mouse, a knockout mouse, or a mouse fed a high-fat diet), or providing other suitable non-human mammals, administering one or more candidate agents to the non-human mammal (*e.g*., treating the mammal with an agent) and determining a biological activity level of the mammal using known assays specific for that biological activity. A comparison of the biological activity level of the mammal that had the agent administered (*e.g*., the treated mammal) to the level of the biological activity of a mammal that did not have the agent administered (a non-treated mammal) is then made. If the biological activity of the agent-treated mammal is altered from the level of activity of the non-treated mammal, then it can be determined that the agent inhibits FATP1 activity. In particular, the biological activities to be measured are glucose uptake glucose clearance, muscle fatty acid accumulation (*e.g*., triglyceride accumulation) fatty acid transport and FATP1-mediated acyl-CoA synthetase activity.

As used herein, the term "inducing insulin resistance" can encompass known methods that trigger insulin resistance such as feeding a mammal a high fat diet for time sufficient to induce insulin resistance, or the use of lipid infusion to induce insulin resistance. Also encompassed in the methods of the invention are insulin resistant mammals obtained from known sources or created by methods known in the art providing, for example, a mammal that has been induced into insulin resistance by knocking out, or disrupting, the FATP1 gene. The production of such mammals is described herein.

The test agent (also referred to herein as the candidate agent) can be administered to the mammal prior to, after or substantially simultaneously with the induction of insulin resistance.

More specifically the present invention covers methods for identifying an agent that inhibits FATP1 activity using a mammal comprising a wild-type FATP1 gene and a mammal comprising a disrupted FATP1 gene. The methods comprise the inducing insulin resistance in the mammal comprising a wild-type FATP1 gene and providing a mammal with a genome comprising a disrupted FATP1 gene; administering a candidate agent to the mammals and determining a biological activity level in the mammals and comparing the biological activity levels of the two mammals wherein the if the biological activity of the mammal whose genome comprises a wild-type FATP1 gene is substantially similar (*e.g*., comparable) to the activity of the mammal whose genome comprises a disrupted FATP 1 gene, then it can be determined that the agent inhibits FATP1. As described above, the biological activity can include for example, glucose uptake, glucose clearance, muscle fatty acid accumulation (*e.g*., triglycende accumulation) fatty acid transport and FATP1-mediated acyl-CoA synthetase activity.

In another embodiment, the invention is directed to a method for identifying an agent that inhibits an FATP1 activity comprising: determining the baseline level of a biological activity associated with the FATP1 activity in a first insulin resistant mouse, wherein the genome of the first mouse comprises a wild-type FATP1 gene; administering to the first and a second mouse whose genome comprises a disrupted FATP1 gene, a test agent; administering to the first and the second mouse an amount of insulin sufficient to allow for the biological activity in a non-insulin resistant mouse; determining the level of the biological activity in the first and second mouse after administering insulin; comparing the level of the biological activity of the first mouse after administering insulin to the baseline level of the biological activity. whereby if the level of the biological activity is altered after administering insulin in the presence of the agent, then the agent modulates the biological activity associated with a FATP1 activity; and comparing the level of biological activity of the second mouse in the presence and absence of the test agent, whereby if the level of the biological activity remains substantially unchanged in the presence and absence of the test agent in the second mouse after administering insulin, then the agent is an inhibitor of FATP1. In particular embodiments, this method is directed to identifying agents that inhibit FATP1-mediated fatty acid transport activity and/or FATP1 acyl-CoA synthetase activity. In one embodiment, the test agent is administered prior to, after or substantially simultaneously with insulin. The biological activity can be, for example, cellular fatty acid accumulation, glucose uptake or glucose clearance. The mouse genome can comprises a homozygous disruption or a heterozygous disruption of the FATP1 gene. In a particular embodiment, insulin resistance is induced in the mouse by a high fat diet or by lipid infusion.

In one embodiment, the invention is directed to a method for identifying an agent useful for the treatment of insulin resistance comprising: administering an amount of insulin and a test agent to a mammal, wherein the test agent binds to mammalian FATP1; determining the amount of glucose uptake in the mamma] administered the test agent; and comparing the amount of glucose uptake in the mammal administered the test agent to the amount of glucose uptake in a mammal administered the same amount of insulin and glucose in the absence of the test agent; wherein a test agent that increases glucose uptake is an agent useful for treatment of insulin resistance. In other embodiments, the method is directed to identifying an agent useful for treatment of non-insulin dependent diabetes mellitus, insulin resistance and/or modulation of cellular triglyceride accumulation.

The methods of the invention encompass determining the acyl-CoA synthetase activity of mammalian FATP1 by measuring the production of a compound selected from the group consisting of phosphate, fatty acyl-CoA and AMP, In other embodiments, the acyl-CoA synthetase activity of mammalian FATP1 is determined by measuring the consumption of a compound selected from the group consisting of coenzyme A, ATP, fatty acid and cholesterol. Additionally, methods can comprise the steps of: administering a candidate agent identified as modulating FATP1 acyl-CoA synthetase activity; and determining whether the candidate agent increases glucose uptake, wherein a candidate agent that increases glucose uptake is useful for treatment of insulm resistance.

In the above methods, insulin can also be administered to the mammal at a time either prior to, after, or substantially simultaneously with the agent. Also in the above described methods, typically the mammal is a mouse. The genomic disruption of the FATP1 gene can be either homozygous or heterozygous.

A further embodiment of the present invention are methods for identifying a candidate agent useful for the treatment of an FATP1-mediated metabolic disorder conaprising, contacting a test agent with a composition comprising mammalian FATP1; measuring the activity of FATP1 the presence of the test agent, comparing the level of FATP1 activity in the presence of the test agent to the level of FATP1 activity in the absence of the test agent; and identifying the agent as a candidate agent useful for the treatment of an FATP1-mediated metabolic disorder where the level of the FATP1 activity is altered in the presence of the test agent. In this embodiment the FATP1 activity measured is selected from the group consisting of acyl-CoA synthetase enzyme activity, fatty acid transport, glucose clearance and glucose uptake; and the FATP1-mediated disorder is selected from the group consisting of insulin resistance, non-insulin dependent diabetes mellitus, and muscle triglyceride accumulation.

In these embodiments, the composition comprising mammalian FATP1 is selected from the group consisting of: purified FATP1 polypeptide, membrane preparations comprising FATP1, and cells comprising FATP1. The activity of FATP1 can be measured by suitable assays known to those of skill in the art, and in particular include an assay selected from the group consisting of: an *in vitro* acyl-CoA synthetase enzyme assay, a cell-based fatty acid transport assay, a tissue-based fatty acid transport assay, or an *in vivo* assay,

The acyl-CoA synthetase activity of FATP1 can be determined by measuring the production of a product selected from the group consisting of phosphate, fatty acyl-CoA and AMP, or by measuring consumption of a substrate selected from the group consisting of coenzyme A, ATP, and fatty acid. Such methods are described herein and are also well-known to those of skill in the art.

The above methods can further comprise administering the identified candidate agent to a mammal having insulin resistance; determining a level of FATP1-mediated biological activity of the mammal; comparing the biological activity of the mammal administered the agent with the biological activity of a mammal not administered agent, and identifying the agent as an agent useful for the treatment of an FATP1-mediated metabolic disorders when the biological activity of the mammal administered agent is altered from the biological activity of the mammal whose not administered agent. The biological activity measured is selected from the group consisting of glucose uptake, glucose clearance, muscle triglyceride accumulation, fatty acid transport, and FATP1-mediated acyl-CoA synthetase activity; and the FATP1-mediated disorder is selected from the group consisting of insulin resistance, non-insulin dependent diabetes mellitus, and muscle triglyceride accumulation.

Also included in the present invention are methods of increasing insulin stimulated whole body glucose uptake in an individual comprising administering to the individual an effective amount of an agent that inhibits FATP1 activity; decreasing blood glucose in an individual comprising administering to the individual an effective amount of an agent that inhibits FATP1 activity; reducing insulin resistance in an individual comprising administering to the individual an effective amount of an agent that inhibits FATP1 activity; reducing triglyceride accumulation in the muscle cells of an individual comprising administering to the individual an effective amount of an agent that inhibits FATP1 activity; and treating diabetes (e.g., non-insulin dependent diabetes mellitus) in an individual comprising administering to the individual an effective amount of an agent that inhibits FATP1 activity.

As described above, the test agent is administered prior to, after, or substantially simultaneously with inducing insulin resistance, and further insulin can be administered to the mammal at a time selected from any of prior to, after, or simultaneously with the agent.

Further embodiments of the present invention include a method of modulating an FATP1-mediated disorder in an individual comprising administering to the individual an effective amount of an agent that inhibits FATP1 activity. In these methods the modulation of an FATP1-mediated disorder comprises any one of the following responses: reducing insulin resistance; increasing insulin stimulated whole body glucose uptake; decreasing blood glucose; reducing muscle triglyceride accumulation, or treating non-insulin dependent diabetes mellitus m an individual.

Thus, the present invention relates to methods of treatment or prevention of conditions (*e.g*., hyperglycemia) or diseases (*e.g*., Type 2 diabetes) associated with insulin resistance. These methods utilize the improvement, *i.e*., decrease or reduction in insulin resistance provided by the inhibition of FATP1-mediated fatty acid transport. In one embodiment the invention provides a method of increasing insulin-stimulated whole body glucose uptake in an individual comprising administering to the individual an agent that inhibits FATP1 activity. Such increases can be exhibited in muscle cells, *e.g*., skeletal muscle cells, or liver cells, or both. In another embodiment, the invention provides a method of decreasing blood glucose in an individual comprising administering to the individual an agent that inhibits FATP1 activity. In yet another embodiment, the invention provides a method of decreasing triglyceride accumulation in muscle cells in an individual comprising administering to the individual an agent that inhibits FATP1 activity. The invention further provides a method of treating diabetes (*e.g*., Type 2 diabetes) or alleviating one or more symptoms clinically associated with diabetes in an individual comprising administering to the individual an agent that inhibits FATP1 activity. The invention further provides a method of preventing the development of diabetes (*e.g*., Type 2 diabetes) in an individual comprising administering to the individual an agent that inhibits FATP1 activity

In one embodiment, the invention is directed to a method for identifying an agent useful for the treatment of insulin resistance comprising administering sufficient glucose to stimulate insulin production and a test agent to a mammal, wherein the test agent binds to mammalian FATP1; determining the amount of glucose uptake in the mammal administered the test agent; and comparing the amount of glucose uptake in the mammal
administered the test agent to the amount of glucose uptake in the mammal administered the same amount of insulin and glucose in the absence of the test agent; wherein a test agent that increases glucose uptake is an agent useful for treatment of insulin resistance.

In another embodiment, the invention is directed to a method for identifying an agent useful for treatment of non-insulin dependent diabetes mellitus comprising determining the amount of glucose uptake in the mammal administered the test agent; and comparing the amount of glucose uptake in the mammal administered the test agent to the amount of glucose uptake in the mammal administered the same amount of insulin and glucose in the absence of the test agent; wherein a test agent that increases glucose uptake is an agent useful for treatment of non-insulin dependent diabetes mellitus.

In yet another embodiment, the invention is directed to a method for identifying an agent useful for modulation of muscle triglyceride accumulation comprising administering a test agent to a mammal, wherein the test agent binds to mammalian FATP1; determining the amount of muscle triglyceride accumulation in the mammal administered the test agent; and comparing the amount of triglyceride in the mammal after administration of the test agent to the amount of triglyceride in the mammal prior to administration of the test agent, wherein when the amount of triglyceride differs after administration of the test agent, an agent useful for the modulation of muscle triglyceride accumulation has been identified. In a particular embodiment, the amount of triglyceride decreases after administration of the agent.

Applicants have further determined that an acyl-CoA synthetase activity is associated with FATP1, and further, that modulation of this activity can result in a decrease in insulin resistance. In another embodiment, the invention is directed to a method for identifying an agent useful for the treatment of insulin resistance comprising contacting a test agent with a mammalian FATP1; determining the level of acyl-CoA synthetase activity of the mammalian FATP1 in the presence of the test agent; and comparing the level of acyl-CoA synthetase activity associated with the mammalian FATP1 in the presence of the test agent to the level of acyl-CoA synthetase activity of the mammalian FATP1 in the absence of the test agent; wherein when the level of the acyl-CoA synthetase activity is decreased in the presence of the test agent, an agent useful for the treatment of insulin resistance has been identified. In a particular embodiment, the acyl-CoA synthetase activity of mammalian FATP1 is determined by measuring the production of a compound selected from the group of phosphate, fatty acyl-CoA and AMP. In alternative embodiments, the acyl-CoA synthetase activity of mammalian FATP1 is determined by measuring the consumption of a compound selected from the group of coenzyme A, ATP and fatty acid In a particular embodiment, the method further comprises: administering a candidate agent identified as modulating FATP1 acyl-CoA synthetase activity; and determining whether the candidate agent increases glucose uptake, wherein a candidate agent that increases glucose uptake is useful for treatment of insulin resistance.

In another embodiment, the invention is directed to a method for identifying an agent useful for the treatment of non-insulin dependent diabetes comprising contacting a test agent with a mammalian FATP1; determining the level of acyl-CoA synthetase activity of the mammalian FATP1 in the presence of the test agent; and comparing the level of acyl-CoA synthetase activity of the mammalian FATP1 in the presence of the test agent to the level of acyl-CoA synthetase activity of the mammalian FATP1 in the absence of the test agent; wherein when the level of the acyl-CoA synthetase activity is decreased in the presence of the test agent, an agent useful for the treatment of non-insulin dependent diabetes has been identified. In particular embodiments, the acyl-CoA synthetase activity of mammalian FATP1 is determined by measuring the production of a compound selected from the group of phosphate, fatty acyl-CoA and AMP. In alternative embodiments, the acyl-CoA synthetase activity of mammalian FATP1 is determined by measuring the consumption of a compound selected from the group of coenzyme A, ATP and fatty acid. In a particular embodiment, the method further comprises administering a candidate agent identified as modulating FATP1 acyl-CoA synthetase activity; and determining whether the candidate agent increases glucose uptake, wherein a candidate agent that increases glucose uptake is useful for treatment of non-insulin dependent diabetes mellitus.

In yet another embodiment, the invention is directed to a method for identifying an agent useful for modulation of muscle triglyceride accumulation comprising contacting a test agent with a mammalian FATP1; determining the level of acyl-CoA synthetase activity of the mammalian FATP1 in the presence of the test agent; and comparing the level of acyl-CoA synthetase activity of the mammalian FATP1 in the presence of the test agent to the level of acyl-CoA synthetase activity of the mammalian FATP1 in the absence of the test agent; wherein when the level of the acyl-CoA synthetase activity differs m the presence of the test agent, an agent useful for the modulation of muscle triglyceride accumulation has been identified. In a particular embodiment, the acyl-CoA synthetase activity is decreased in the presence of the agent. In particular embodiments, the acyl-CoA synthetase activity of mammalian FATP1 is determined by measuring the production of a compound selected from the group of phosphate, fatty acyl-CoA and AMP. In alternative embodiments, the acyl-CoA synthetase activity of mammalian FATP1 is determined by measuring the consumption of a compound selected from the group of coenzyme A, ATP, fatty acid and cholesterol. In a particular embodiment, the method further comprises: administering a candidate agent identified as modulating FATP1 acyl-CoA synthetase activity; and determining whether the candidate agent modulates muscle triglyceride accumulation, thereby ideptifying an agent useful in modulating triglyceride accumulation.

Thus, the present invention provides a source of cells, animals and methods useful for elucidating the function of FATP1 in intact animals whose genomes comprise a wild-type FATP1 gene and for the identification of agents , *e.g.,* therapeutic agents, that inhibit FATP1 activity; and methods of treating diseases or conditions associated with FATP1 function. Such diseases or conditions include insulin resistance, non-insulin dependent diabetes mellitus, and muscle triglyceride accumulation.

### BRIEF DESCRIPTION OF THE DRAWINGS

FIG. 1 is a schematic illustrating the vector used to disrupt the function of the wild-type munne FATP1 gene in the FATP1 knockout mice.
FIG. 2 is a bar graph illustrating the results of experiments comparing the rate of glucose disappearance between groups of wild-type mice and FATP1 knockout mice at basal levels, mice infused with lipids for five hours and mice fed a high fat diet for three weeks.
FIG. 3 is a bar graph illustrating the results of experiments comparing glucose transport in gastrocneumius muscle between wild-type mice and FATP1 knockout mice at basal levels, mice infused with lipids for five hours and mice fed a high fat diet for three weeks.
FIG. 4 is a bar graph illustrating the results of experiments comparing glucose transport in white adipose tissue (WAT) between wild-type mice and FATP1 knockout mice at basal levels, mice infused lipids for five hours and mice fed a high fat diet for three weeks.
FIG, 5 is a bar graph illustrating the results of experiments comparing the level of triglycende (TG) in quadriceps muscle between wild-type mice and FATP1 knockout mice at basal levels, mice infused with lipids for five hours and mice fed a high fat diet for three weeks.
FIG. 6 is a bar graph illustrating the results of experiments comparing the level of triglyceride (TG) in liver between wild-type mice and FATP1 knockout mice at basal levels, mice infused with lipids for five hours and mice fed a high fat diet for three weeks.
FIG. 7A is a bar graph comparing the body weights of wild-type mice, heterozygous mice, and FATP1 knockout mice fed standard diets and those fed a high fat diet. FIG. 7B is a bar graph comparing the fat/lean mass of wild-type mice fed standard diets to wild-type and FATP1 knockout mice fed high fat diets.
FIG. 8 is a graph comparing the results of glucose tolerance tests performed on wild-type mice fed standard diets and wild-type and FATP1 knockout mice fed high fat diets. The amount of glucose in mg/dl is compared to the rate of clearance in minutes.

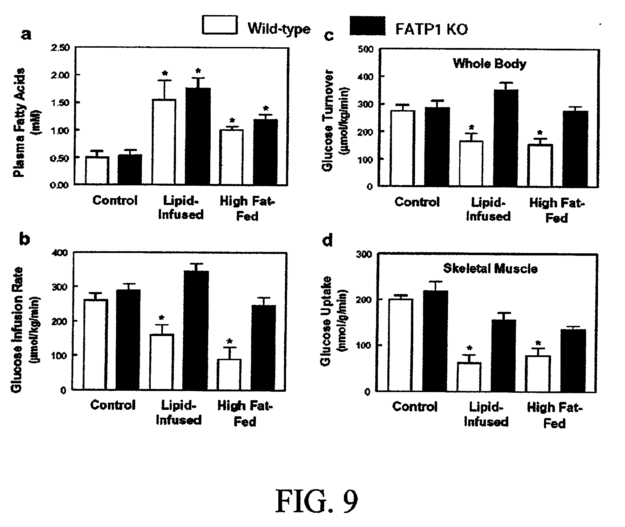

FIGS. 9A-9D are graphs showing plasma fatty acids concentrations and insulin-stimulated whole body and skeletal muscle (gastrocnemius) glucose uptake in the wild-type and FATP1 knockout mice with lipid infusion or high far feeding. FIG. 9A: Plasma fatty acids concentrations. FIG. 9B: Steady state glucose infusion rate, obtained from averaged rates of 90 to 120 minutes of hyperinsulinemic-euglycemic clamps. FIG. 9C: Insulin-stimulated whole body glucose turnover *in vivo.* FIG. 9D: Insulin-stimulated skeletal muscle glucose uptake *in vivo.* Values are means ± S.E.M. for 5∼6 experiments. *P < 0.05 vs. wild-type (control) mice.
FIGS. 10A-10D are graphs showing insulin-stimulated whole body and skeletal muscle (gastrocnemius) glucose metabolic flux in the wild-type and FATP1 knockout mice with lipid infusion or high far feeding, FIG. 10A. Insulin-stimulated whole body glycolysis *in vivo.* FIG. 10B: Insulin-stimulated whole body glycogen/lipid synthesis *in vivo*. FIG. 10B: Insulin-stimulated skeletal muscle glycolysis *in vivo.* FIG. 10D: Insulin-stimulated skeletal muscle glycogen synthesis *in vivo.* Values are means ± S.E.M. for 5∼6 experiments. *P < 0.05 vs. wild-type (control) mice.
FIGS. 11A-11D are graphs showing insulin signaling and fat metabolite concentrations in skeletal muscle (gastrocnemius and quadriceps, respectively) of the wild-type and FATP1 knockout mice with lipid infusion or high fat feeding. FIG. 11A: Insulin-stimulated tyrosine phosphorylation of IRS-1. FIG. 11B: Insulin-stimulated IRS-1 associated PI 3-kinase activity. FIG. 11D: Intramuscular triglyceride concentrations. FIG. 11D: Intramuscular fatty acyl-CoA concentrations. Values are means ± S.E.M. for 5∼6 experiments. *P < 0.05 vs. wild-type (control) mice.

### DETAILED DESCRIPTION OF THE INVENTION

The present invention provides methods for identifying an agent that modulates FATP-mediated fatty acid transport activity. The findings described herein demonstrate that inhibition of FATP1-mediated fatty acid transport leads to a reduction of insulin resistance, a characteristic of Type 2 diabetes mellitus, and a reduction in insulin-stimulated glucose uptake, indicate that an agent that inhibits FATP1-mediated fatty acid transport are useful in reducing insulin resistance, and, therefore, useful for treating Type 2 diabetes mellitus.

The present invention is based on the discovery that, in FATP1 knockout mice, insulin resistance is reduced. The reduction in insulin resistance allows for a higher level of glucose metabolism. FATP1 was isolated in a screen for putative fatty acid transporters (Shaffer, J. and Lodish, H., 1994, *Cell*, 79:427-436). Subsequently, FATP1 has been shown to have an acyl-CoA synthetase activity (Choi, J. and Martin, C., 1999. *J. Biol Chem*., 274:4671-4683). Described herein are data that show the accumulation of modified fatty acids in muscle and liver tissue or cells does not occur in the absence of FATP1. While not wishing to be bound by theory, the activities of FATP1 suggest details as to how insulin resistance is established and support the validity of FATP1 as a therapeutic target for use in insulin resistant related disorders, including Type 2 diabetes mellitus, among others.. As Type 2 diabetes mellitus is characterized by insulin resistance and a low level of glucose metabolism, the resulis described herein allow for methods of treating Type 2 diabetes mellitus as well as the associated phenotypes such as, for example. decreased glucose metabolism, insulin resistance and cellular fatty acid accumulation. The invention describes mice that lack functional FATP1, methods of using such mice, methods of using cells derived from such mice, methods of using cells lacking or partially lacking FATP1 activity, in vitro methods for identifying or evaluating agents that modulate FATP1 activity, and methods for using identified agents for treating FATP 1-mediated metabolic disorders (*e.g*., Type 2 diabetes mellitus, insulin resistance, and cellular triglyceride accumulation).

The mechanism by which increased levels of fatty acid metabolites cause muscle insulin resistance involves activation of serine kinase cascade, of which protein kinase C (PKC)-β and/or lκB kinase-β (IKK-β) may play a role, leading to the serine phosphorylation of IRS-1. Recent studies have shown that serine phosphorylation of IRS-1 prevents tyrosine phosphorylation of IRS-1 and interferes with its ability to recruit and activate PI 3-kinase, as occurs upon treatment with TNF-α and okadaic acid (Itani, S. *et al*., 2000. *Diabetes*, 49:1353-1358;. Yuan, M. *et al*., 2001. *Science*, 293:1673-1677; Kim, J. *et al*., 2001. *J. Clin Invest*., 108:437-446, Schmitz-Peiffer, C. *et al*., 1997. *Diabetes,* 46:169-178; Rui, L. *et al*., 2001. *J. Clm. Invest*., 107:181-189; Pederson, T. *et al*., 2001. *Diabetes*, 50:24-31; De Fea, K. and Roth, R., 1997. *Biochemistry*, 36:12939-12947; Tanti, J.-F. *et al., J. Biol. Chem*., 269:6051-6057; Griffin, M. *et al*., 1999. *Diabetes*, 48:1270-1274; Schmitz-Peiffer, C. *et al.,* 1997. *Diabetes,* 46:169-178; Chalkley, S. *et al*., 1998. *Metabolism,* 47:1121-1126).

Other studies have also implicated circulating factors released by adipocytes to play a role in skeletal muscle insulin resistance. Tumor necrosis factor alpha is an adipocyte-derived multifunctional cytokine that has been shown to be elevated in obese animal models (Hofmann, K. *et al*., 1994. *Endocrinology,* 134:264-270; Hotamisligil, G. *et al*., 1993. *Science,* 259:87-91; Hotamisligil, G. *et al*, 1996. *Science,* 271:665-668). Moreover, resistin is another adipocyte-derived circulating factor that has been recently shown to be elevated in insulin resistant high fat-fed mice as well as in ob/ob mice (Steppan, C. *et al*., 2001. *Nature,* 409:307-312). On the other hand, others have shown that resistin expression was not elevated in insulin resistant, obese, or Type 2 diabetic subjects and argued against the role of resistin in obesity-related insulin resistance in humans (Nagaev, I. and Smith, U., 2001. *Biochem. Biophys, Res. Commun*., 285:561-564; Sentinelli, F. *et al*., 2002. *Diabetes,* 51:860-862; Savage, D. *et al., Diabetes*, 50:2199-2202). Additionally, adiponectin/acrp30, a novel adipocyte-specific collagen-like molecule, has been shown to be decreased in obese and Type 2 diabetic subjects(Arita, Y. *et al*., 1999. *Biochem. Biophys Res. Commun*., 257:79-83), Hotta and colleagues have shown a parallel relationship between plasma adiponectin concentrations and progression of insulin resistance, as measured by fasting plasma glucose and insulin concentrations, in rhesus monkeys, while Yamauchi and colleagues demonstrated that adiponectin increased skeletal muscle insulin action (Hotta, K. *et al*., 2001. *Diabetes,* 50:1126-1133; Yamauchi, T. *et al*., 2001. *Nat. Med.,* 7:941-946). While it is clear that circulating adipocyte-denved factors can modulate skeletal muscle insulin action, the present findings that FATP1 knockout mice were protected from fat-induced increases in intramuscular fatty acyl-CoA and insulin resistance, demonstrate an important role for intramuscular fatty acid metabolites as causative agents in the development of insulin resistance in skeletal muscle. These results also implicate FATP1 as a novel therapeutic target in the treatment of insulin resistance and Type 2 diabetes.

The present invention provides a "knockout" or transgenic non-human mammal, *e.g*., a non-human primate, a rodent such as a mouse or rat, a sheep, a dog, a cow, a pig, a rabbit or a goat. As used herein, "knockout" refers to a genetically modified organism that has a genome in which a particular gene has been disrupted or deleted such that expression of the gene is eliminated or occurs at a reduced level.. In particular embodiments, the knockout non-human mammal is a rodent, *e.g.*, a mouse or a rat. For example, a FATP1 knockout mammal comprises disruption of an endogenous FATP1 gene, such that the mammal lacks or has reduced levels of functional FATP1 protein. In a particular embodiment, the non-human knockout mammal is a mouse that lacks a functional FATP1 gene product or exhibits a reduced level of the FATP1 gene product. The transgenic mouse is referred to herein as a "transgenic FATP1 knockout mouse" or a "FATP1 knockout mouse". In a particular embodiment, the genome of the FATP1 knockout mouse comprises at least one non-functional allele for the endogenous FATP1 gene. Thus, the invention provides a source of cells (*e.g*., tissue, cells, cellular extracts, organelles) and animals useful for elucidating the function of FATP1 in intact animals whose genomes comprise a functional and referential, sometimes referred to as a "wild-type", FATP1 allele.

Any suitable mammal can be used to produce the FATP1 knockout mammal described herein. For example, a suitable mammal can be a non-human primate, a sheep, a dog, a cow, a goat, a mouse (mice), a rat, a rabbit or a pig.

As used herein, the term "gene" refers to DNA sequences that encode the genetic information (*e g*., nucleic acid sequence) required for the synthesis of a single protein (*e.g*., polypeptide chain). In addition to the "coding sequence", the sequence that directly codes the ammo acid sequence, a gene also includes essential noncoding elements, *e.g*., promoters, enhancers, silencers, and non-essential flanking and intron sequences. The term "FATP1 gene" refers to a particular mammalian gene that comprises a DNA sequence that encodes the FATP1 protein. As is understood by one of skill in the art, a gene sequence can contain "sites" (sequence positions) that are different among individuals in a population. Thus, a gene allows for variation of the sequence. Each variant sequence is referred to as an "allele" of the gene. Typically, a particular sequence, usually one that encodes a functional protein, is taken to be a reference or "wild-type" sequence; the term "wild-type" is a descriptive term meant to connote a reference allele, typically an allele that encodes a functional protein or an allele present in a healthy individual. Alleles that differ from the wild-type sequence are referred to as "allelic variants". Homologous chromosomes are chromosomes that pair during meiosis and contain substantially identical loci. The term "locus" connotes the site (*e.g*., location) of a gene on a chromosome.

The structures and coding sequences for a number of FATP1 genes have been previously reported. In particular, the mouse and human FATP1 coding sequences have been reported (Schaffer, J. and Lodish, H., 1994. *Cell*, 79:427-436; Stahl, A. *et al*., 1998. *Mol. Cell,* 4:299-308). The mouse coding sequence is reported in GenBank under the Accession Number U159.76. The structure of the human gene (*i.e*. intron-exon boundaries) has also been reported (Martin, G. *et al*., 2000. *Genomics*, 66:296-304). The referenced FATP1 sequences are hereby incorporated by reference.

As used herein the terms "disruption", ''functional inactivation", "alteration" and "defect" connote a partial or complete reduction in the expression and/or function of the FATP1 polypeptide encoded by the endogenous gene of a single type of cell, selected cells or all of the cells of a FATP1 knockout mouse. Thus, according to the instant invention the expression or function of the FATP1 gene product can be completely or partially disrupted or reduced (*e.g*., by 50%, 75%, 80%, 90%, 95% or more) in a selected group of cells (*e.g*., a tissue or organ) or in the entire animal As used herein the term "a functionally disrupted FATP1 gene" includes a modified FATP1 gene that either fails to express any polypeptide product or that expresses a truncated protein having less than the entire amino acid polypeptide chain of a wild-type protein and is non-functional (partially or completely non-functional).

Disruption of the FATP1 gene can be accomplished by a variety of methods known to those of skill in the art. For example, gene targeting using homologous recombination. mutagenesis (*e.g*., point mutation), RNA interference and antisense technology can be used to disrupt a FATP1 gene.

More specifically, the invention provides a knockout mammal, *e.g.* mouse, whose genome comprises either a homozygous or heterozygous disruption of its FATP1 gene. A knockout mammal whose genome comprises a homozygous disruption is characterized by somatic and germ cells that contain two nonfunctional (disrupted) alleles of the FATP1 gene, while a knockout mammal whose genome comprises a heterologous disruption is characterized by somatic and germ cells that contain one wild-type allele and one nonfunctional allele of the FATP1 gene.

As used herein, the term "genotype" refers to the genetic makeup of an animal. A particular genotype refers to one or more specific genes, *e.g*., FATP1. More specifically the term genotype refers to the status of the ammal's FATP1 alleles, which can either be intact and functional (*e.g*., wild-type or +/+); or disrupted (*e.g*., knockout) in a manner that confers either a heterozygous (*e g*., +/-); or homozygous (-/-) knockout genotype.

The present invention also provides methods of producing a non-human mammal that lacks a functional FATP1 gene. Briefly, the standard methodology for producing a knockout embryo requires introducing a targeting construct, which is designed to integrate by homologous recombination with the endogenous nucleic acid sequence of the targeted gene, into a suitable embryonic stem cell (ES). The ES cells are then cultured under conditions that allow for homologous recombination (*i.e*., of the recombinant nucleic acid sequence of the targeting construct and the genomic nucleic acid sequence of the host cell chromosome). Genetically engineered stem cells that are identified as comprising a knockout genotype that comprises the recombinant allele are introduced into an animal, or parent thereof, at an embryonic stage using standard techniques that are well known in the art (*e.g*., by microinjecting the genetically engineered embryonic stem (ES) cell into a blastocyst). The resulting chimeric blastocyst is then placed within the uterus of a pseudopregnant foster mother for the development into viable pups. The resulting viable pups include potentially chimeric founder animals whose somatic and germline tissue comprise a mixture of cells derived from the genetically-engineered ES cells and the recipient blastocyst. The contribution of the genetically altered stem cell to the germline of the resulting chimeric mice allows the altered ES cell genome, which comprises the disrupted target gene, to be transmitted to the progeny of these founder animals, thereby facilitating the production of "knockout ammals" whose genomes comprise a gene that has been genetically engineered to comprise a particular defect in a target gene.

One of skill in the art will easily recognize that the FATP1 gene can be disrupted in a number of different ways, any one of which may be used to produce the FATP1 knockout mammals of the present invention. For example, a knockout mouse according to the instant invention can be produced by the method of gene targeting As used herein the term "gene targeting" refers to a type of homologous recombination that occurs as a consequence of the introduction of a targeting construct (*e g*., vector) into a mammalian cell (*e*.*g*., an ES cell) that is designed to locate and recombine with a corresponding portion of the nucleic acid sequence of the genomic locus targeted for alteration (*e g.,* disruption) thereby introducing an exogenous recombinant nucleic acid sequence capable of conferring a planned alteration to the endogenous gene. Thus, homologous recombination is a process (*e.g*., method) by which a particular DNA sequence can by replaced by an exogenous genetically engineered sequence. More specifically, regions of the targeting vector that have been genetically engineered to be homologous or complementary to the endogenous nucleotide sequence of the gene that is targeted for transgenic disruption line up or recombine with each other such that the nucleotide sequence of the targeting vector is incorporated into (*e.g*, integrates with) the corresponding position of the endogenous gene.

One embodiment of the present invention provides a vector construct (*e.g*., a FATP1 targeting vector or FATP1 targeting construct) designed to disrupt the function of a wild-type (endogenous) FATP1 gene. In general terms, an effective FATP1 targeting vector comprises a recombinant sequence that is effective for homologous recombination with an endogenous FATP1 gene. For example, a replacement targeting vector comprising a genomic nucleotide sequence that is homologous to the target sequence operably linked to a second nucleotide sequence that encodes a selectable marker gene exemplifies an effective targeting vector. integration of the Targeting sequence into the chromosomal DNA of the host cell (*e.g*., embryonic stem cell) as a result of homologous recombination introduces an intentional disruption, defect or alteration (*e.g*., insertion, deletion or substitution) into the targeted sequence of the endogenous gene, *e.g*., the FATP1 gene. One aspect of The present invention is to replace all or part of the nucleotide sequence of a non-human mammalian gene that encodes the FATP1 polypeptide, thereby making a transgenic FATP1 knockout.

One of skill in the art will recognize that any FATP1 genomic nucleotide sequence of appropriate length and composition to facilitate homologous recombination at a specific site that has been preselected for disruption can be employed to construct a FATP1 targeting vector. Guidelines for the selection and use of sequences are described for example in Deng, C. and Capecchi, M., 1992, *Mol. Cell. Biol*., 12:3365-3371, and Bollag, R *et al*., 1989, *Annu. Rev. Genet,* 23:199-225. For example, a wild-type FATP1 gene can be mutated and/or disrupted by inserting a recombinant nucleic acid sequence (*e.g.*, a FATP1 targeting construct or vector) into all or a portion of the FATP1 gene locus. For example, a targeting construct can be designed to recombine with a particular portion within the enhancer, promoter, coding region, start codon, noncoding sequence, introns or exons of the FATP1 gene. Alternatively, a targeting construct can comprise a recombinant nucleic acid that is designed to introduce a stop codon after an exon of the FATP1 gene.

Suitable targeting constructs of the invention can be prepared using standard molecular biology techniques known to those of skill in the art. For example, techniques useful for the preparation of suitable vectors are described by Maniatis, *et al*., Molecular Cloning: A Laboratory Manual, Cold Spring Harbor Laboratory, Cold Spring Harbor, N. Y.; which disclosures are hereby incorporated by reference. Appropriate vectors include a replacement vector such as the insertion vector described by Capecchi, M., 1989, *Science,* 244:1288-92, which disclosure is hereby incorporated by reference; or a vector based on a promoter trap strategy or a polyadenylation trap, or "tag-and-exchange" strategy described by Bradley, *et al*., 1992, *Biotechnology (NY)*, 10:534-539; and Askew, G. *et al*., 1993, *Mol. Cell. Biol.*, *13*:4115-4124, which disclosures are also incorporated herein by reference.

One of skill m the art will readily recognize that a large number of appropriate vectors known in the art can be used as the basis of a suitable targeting vector. In practice, any vector that is capable of accommodating the recombinant nucleic acid sequence required to direct homologous recpmbination and to disrupt the target gene can be used. For example, pBR322, pACY164, pKK223-3, pUC8, pKG, pUC19, pLG339, pR290, pKC101 or other plasmid vectors can be used. Alternatively, a viral vector such as the lambda gr11 vector system can provide the backbone (*e.g*. cassette) for the targeting construct.

According to techniques well known to those of skill in the art genetically engineered (*e.g*., transfected using electroporation or transformed by infection) embryonic stem cells are routinely employed for the production of transgenic and knockout non-human embryos. Embryonic stem (ES) cells are plunpotent cells isolated from the inner cell mass of a mammalian blastocyst. ES cells can be cultured *in vitro* under appropriate culture conditions in an undifferentiated state and retain the ability to resume normal *in vivo* development and differentiation as a result of being combined with a blastocyst and introduced into the uterus of a pseudopregnant foster mother. Those of skill in the art will recognize that various stem cells are known in the art, for example AB-1, HM-1, D3. CC1.2, E-14T62a, RW4 or JI (Teratomacarcinoma and Embryonic Stem Cells: A Practical Approach, E. J. Roberston, ed., IRL Press).

It is to be understood that the FATP1 knockout mammals described herein can be produced by methods other than the embryonic stem cell method described above, for example, by the pronuclear injection of recombinant genes into the pronuclei of one-cell or transformed embryos or other gene targeting methods that do not rely on the use of a transfected or transformed ES cell, and that the exemplification of the single method outlined above is not intended to limit the scope of the invention to animals produced solely by this protocol.

The FATP1 knockout mammals described herein can also be bred (*e.g,* inbred, outbred or crossbred) with appropriate mates to produce colonies of animals whose genomes comprise at least one non-functional allele of the endogenous gene that naturally encodes and expresses functional FATP1 Examples of such breeding strategies include but are not limited to: crossing of heterozygous knockout animals To produce homozygous animals; outbreeding of founder animals (*e.g*., heterozygous or homozygous knockouts) with a mouse whose inbred genetic background confers aberrant insulin and/or glucose homeostasis or that provides an animal model of diabetes and crossbreeding a founder animal with an independent transgenic animal that has been genetically engineered to overexpress a gene associated with increased susceptibility to diabetes and/or obesity. For example, a founder knockout mouse could be bred with the *ob*/*ob* mouse, the *db*/*db* mouse and/or the AY mouse.

The FATP1 knockout mammals, *e.g*., mice, are useful for a variety of purposes. In one embodiment, a FATP1 knockout cell or cell line can be engineered using skills known in the art. For example, cells that do not possess an endogenous FATP1 gene or that normally do not express FATP1 can be engineered to do so. For example, an exogenous FATP1 gene can be introduced into a cell that does not possess an endogenous FATP1 gene wherein the cell expresses FATP1 due to the presence of the exogenous FATP1 gene. Alternatively, exogenous nucleic acid can be spliced into the genome of a cell that does not normally express FATP1 in order to "turn on" the normally silent FATP1 gene. The agent can be for example, a nucleic acid molecule, a polypeptide, an organic molecule, an inorganic molecule, a fusion protein *etc*. Subsequently the FATP1 gene in the engineered cells can be disrupted using the methods described herein and known to those of skill in the art for use in the methods and compositions of the present invention.

As a result of the disruption of the FATP1 gene, the FATP1 knockout mouse of the present invention can manifest a particular phenotype The term phenotype refers to the resulting biochemical or physiological consequences attributed to a particular genotype. In the situation where a knockout mouse has been created, the phenotype observed is a result of the loss of the gene that has been knocked out. In one embodiment, the FATP1 knockout mouse has altered insulin/glucose homeostasis and responsiveness to glucose stimulation. The FATP1 knockout mouse has an increased responsiveness to glucose stimulation, and, as a result, exhibits increased glucose uptake when compared to the glucose uptake exhibited by an appropriate control mouse (*e.g.*, wild-type) in response to the same glucose challenge. FATP1 knockout mice also exhibit reduced insulin resistance. In addition, FATP1 knockout mice exhibit reduced modified fatty acid (triglyceride) accumulation in muscle Specifically, acyl-CoA-modified muscle triglyceride accumulation is reduced in FATP1 knockout mice relative to wild-type mice. Further aspects of the invention provide a method for the identification of agents (*e.g.,* therapeutic agents) that modulate, *e.g*., increase or decrease, FATP1 function; and a method of treating diseases or conditions associated with FATP1 function (*e.g*., hyperglycemia, insulin resistance, Type 2 diabetes).

In addition, Applicants have discovered that FATP1-mediated transport of fatty acids significantly affects insulin resistance. It has been demonstrated that increased fatty acid delivery to muscle induces muscle-specific insulin resistance. As used herein, the term "inducing insulin resistance'' can encompass known methods that trigger insulin resistance such as feeding a mammal a high fat diet for time sufficient to induce insulin resistance, or the use of lipid infusion to induce insulin resistance. Insulin resistance can also be induced by knocking out genes such that insulin resistance is triggered. The production of such mammals is described herein. It has also been demonstrated that increased fatty acid delivery to liver causes liver-specific insulin resistance. It has now been determined that inhibition of FATP1-mediated transport of fatty acids significantly reduces "insulin resistance", a reduced biological response to either exogenous or endogenous insulin. Insulin resistance is a characteristic of Type 2 diabetes mellitus and leads to the inability of the affected individual to metabolize glucose, *e.g*., clear glucose from the blood stream and take up glucose into cells

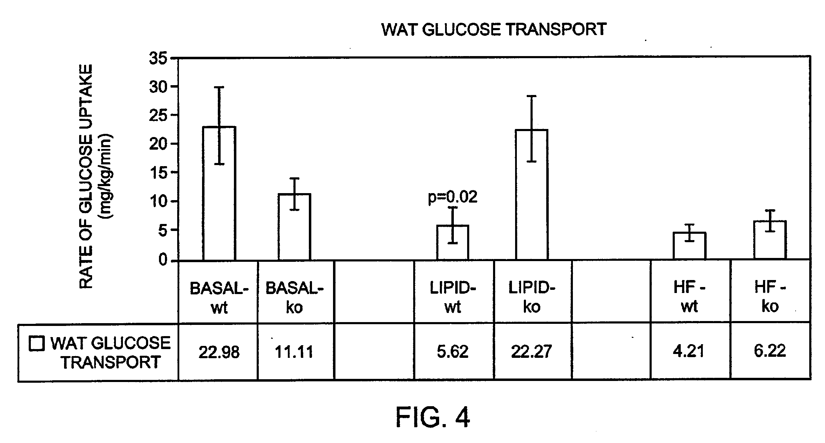

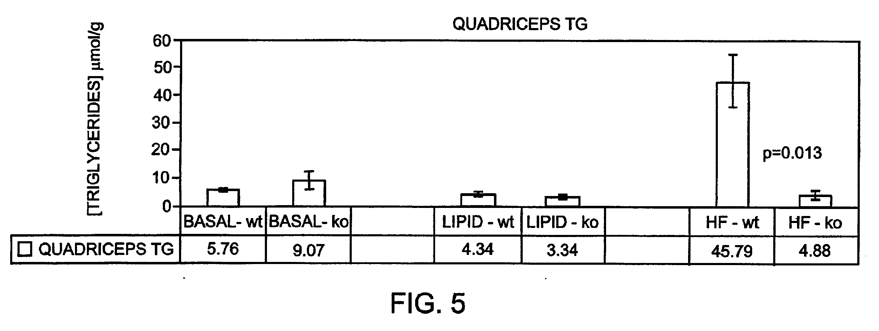

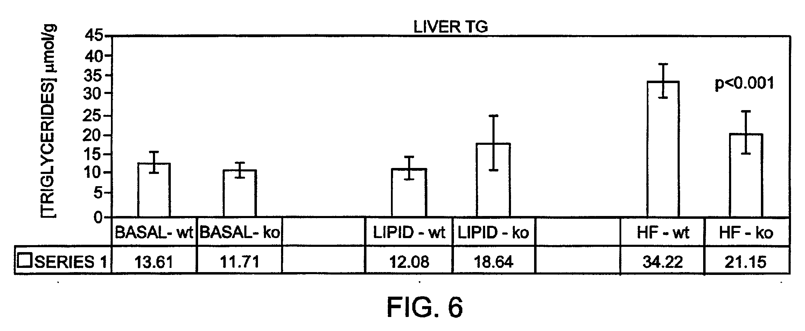

Although not wishing to be bound by theory, the mechanism by which FATP1 deletion protects from lipid-induced and high-fat diet induced insulin resistance can be proposed from its function mediating fatty acid transport. In particular, absence of FATP1 from muscle and adipose tissue (tissues in which FATP1 is most highly expressed) leads to decreased fatty acid uptake into these tissues. As a consequence, fatty acid derivatives, which accumulate in these tissues as a result of lipid infusion or high-fat diet, cannot accumulate, thus eliminating the induction of insulin resistance. Consistent with this hypothesis, high-fat diet induced triglyceride accumulation in muscle is completely abolished in FATP1 knockout mice (see FIG. 5). Interestingly, high-fat diet induced triglyceride accumulation in white adipose tissue is not altered by FATP1 deletion, consistent with a lack of protection from insulin resistance in the white adipose tissue on a high fat diet (see FIG. 4). High-fat diet-induced triglyceride accumulation in the liver is partially reversed in the FATP1 deletion mice (see FIG. 6), suggesting that FATP1 may also play a role in fatty acid uptake in the liver. Under conditions of acute lipid infusion, no elevation of cellular triglycerides can be seen, but the resultant elevation of fatty acid metabolites may be the cause of insulin resistance,

FATP1 has also been shown to possess an acyl-CoA synthetase activity. The fact that FATP1 facilitates transport of acyl-CoA-modified triglycerides and has an acyl-CoA synthetase activity suggests that the enzymatic and transport activities are related. Acyl-CoA synthetase activity can be measured in the absence of transport to characterize enzymatic activity, for example, using purified or partially purified proteins, cellular membrane fractions, cellular lysates or other sources of FATP1 suitable for *in vitro* enzymatic assays (*e.g*., monitoring the depletion of starting materials such as coenzyme A, ATP and fatty acids, or the release of phosphate, acyl-CoA-modified fatty acids and AMP). Methods for expressing and purifying proteins are well known in the art. While not being bound by theory, the accumulation of acyl-CoA-modified triglycerides in muscle or liver can lead to or can serve as an indicator of insulin resistance. An animal, tissue or cell that is insulin resistant has a reduced ability to respond to an insulin signal to take up glucose. Thus, the data presented herein identify a therapeutic target molecule for treating insulin resistance and Type 2 diabetes mellitus, namely, FATP1, Inhibition of FATP1 reduces modified fatty acid uptake and insalin resistance, and increases glucose uptake. The invention therefore is directed, in part, to methods for identifying an agent that inhibits FATP1 for use in treating Type 2 diabetes mellitus, insulin resistance, improving glucose metabolism and/or reducing fatty acid accumulation in muscle.

FATP1 facilitates the transport of modified fatty acids (triglycerides) into cells (*e.g*., adipose, muscle and heart, where FATP1 is predominantly expressed). Therefore, inhibition of FATP1-mediated fatty acid transport reduces the amount of triglycerides incorporated into cells that normally express FATP1. FATP1 has been shown to possess an acyl-CoA synthetase activity. The identification of an agent that modulates or alters an FATP1 activity, *e.g*., FATP1-mediated fatty acid transport or acyl-CoA synthetase activity, involves measuring the FATP1 activity before and after administering a test agent and comparing the levels of The activity in the presence and absence of the test agent. Methods described below and known in the art for determining the level of an FATP1-associated activity, *e.g*., acyl-CoA synthetase activity and fatty acid transport, are appropriate for the methods of the present invention

FATP1-mediated fatty acid transport and FATP1 acyl-CoA synthetase activity can be measured *in vivo, ex vivo* and/or *in vitro.* FATP1-mediated fatty acid transport activity can be measured, for example, by determining the level (used herein to refer to either amount or rate) of acyl-CoA-modified fatty acid accumulation in cells (*e.g*., muscle, heart, liver or adipose tissue cells), glucose uptake or glucose clearance. Acyl-CoA synthetase activity can be measured, for example, by determining the level of acyl-CoA-modified fatty acid accumulation in muscle; consumption of coenzyme A, ATP, fatty acid and/or cholesterol; and/or production of phosphate, fatty acyl-CoA or AMP (see examples 7-9 and 14).

As illustrated in FIG, 5, experiments were conducted to measure triglyceride accumulation in muscle tissue, *e.g*, quadriceps muscle, between wild-type mice and knockout mice. A group of wild-type mice and a group of FATP1 knockout mice were infused with lipids, while a second group of wild-type mice and a second group of FATP1 knockout mice were fed high fat diets for a longer period of time. The respective levels of muscle triglyceride accumulation in each group were compared to the baseline levels determmed for each type of mouse. The wild-type mice showed a ten-fold increase in muscle triglyceride when fed a high-far diet, while FATP1 knockout mice were completely protected from triglyceride accumulation in the muscle when fed the same diet.

FATP1-mediated fatty acid transport can also be determined by monitoring the level of glucose uptake or glucose clearance in animals, tissues or cultured cells (*e.g*., adipose, muscle, heart or transgenic cell lines). As FATP1-mediated fatty acid transport and glucose metabolism are inversely related, *i.e*., an increase in fatty acid transport leads to a reduction in glucose metabolism, measuring glucose metabolism, *e.g*., glucose uptake or glucose clearance, is a measure of FATP1-mediated fatty acid transport. This measurement of determining FATP1-mediated fatty acid transport activity is based on the finding described herein that an FATP1 knockout mouse exhibits a reduced insulin resistance (see Example 10), and, therefore, an improved ability to take up glucose (or clear glucose). Such improvement is demonstrated, for example, by an increase in insulin-stimulated whole body glucose uptake or by a reduction of circulating glucose levels. Glucose metabolism can be measured, for example, by glucose clearance (see Example 13) or glucose uptake (see Example 4).

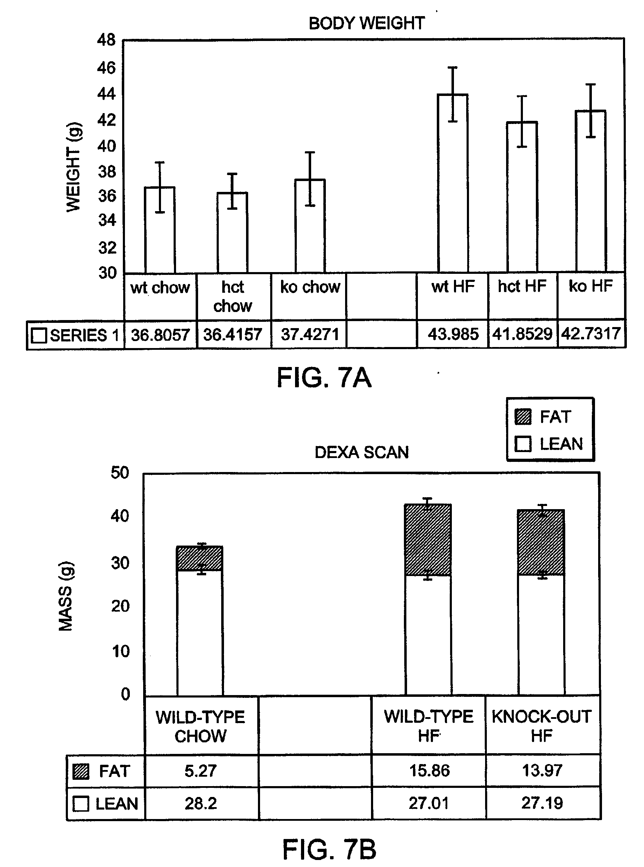

Experimental work provided herein indicates that the altered glucose homeostasis exhibited by the knockout mice of the instant invention is independent of the level of obesity. As illustrated in FIG. 7A, wild-type mice, heterozygous mice and FATP1 knockout mice exhibited significant weight gain on high fat diets. This indicates that the improvement in insulin resistance is independent of the obesity factor. Similarly, as illustrated in FIG. 7B, the ratio of fat tissue to lean tissue increased in both wild-type and FATP1 knockout mice fed high fat diets.

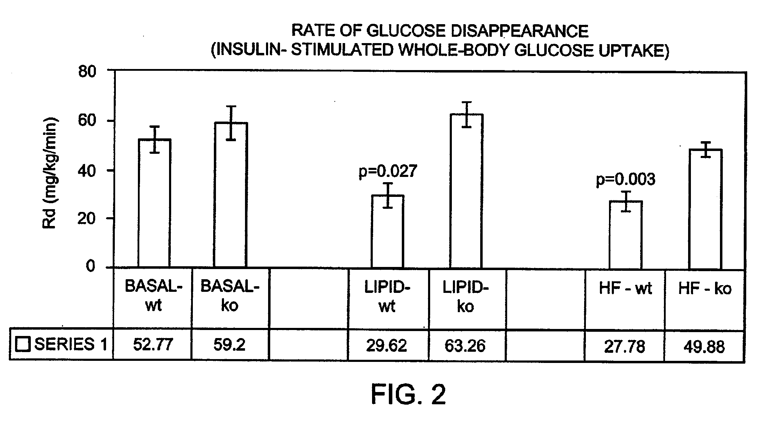

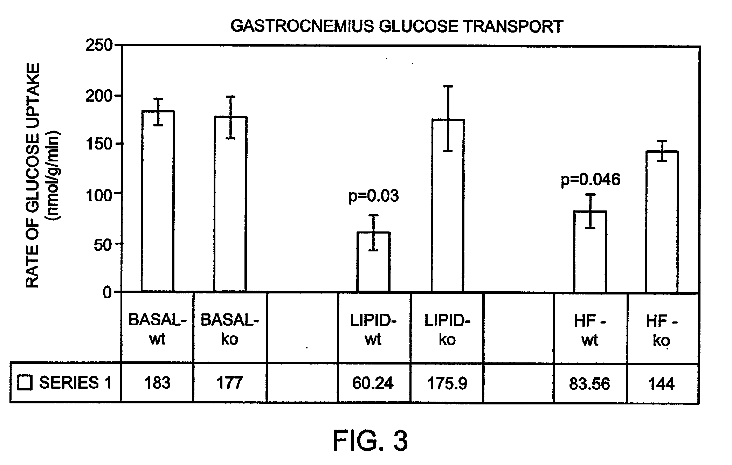

FIG. 2 illustrates the results of experiments conducted to compare the rate of glucose disappearance, and thus, the rate of insulin-stimulated whole body glucose uptake between wild-type mice and knockout mice. To induce insulin resistance, a group of wild-type mice and a group of FATP1 knockout mice were infused with lipids, while a second group of wild-type mice and a second group of FATP1 knockout mice were fed high fat diets for a longer period of time. While the level of whole body glucose disappearance decreased in wild-type mice infused with lipids or fed a high-fat diet, indicating the development of insulin-resistance, FATP1 knock-out mice were completely protected from the effects of lipid infusion and high-fat diet, showing normal levels of glucose disappearance, indicating protection from insulin resistance by FATP1 deletion. Similar results were also achieved when glucose transport was measured in the gastrocnemius muscle (see FIG 3), indicating that muscle may be the primary site for protection from insulin-resistance in FATP1 knock-out mice. FATP1 knockout mice were also protected from insulin resistance in the white adipose tissue if insulin-resistance was induced by lipid infusion, but not if it was induced by high-fat diet (see FIG. 4).

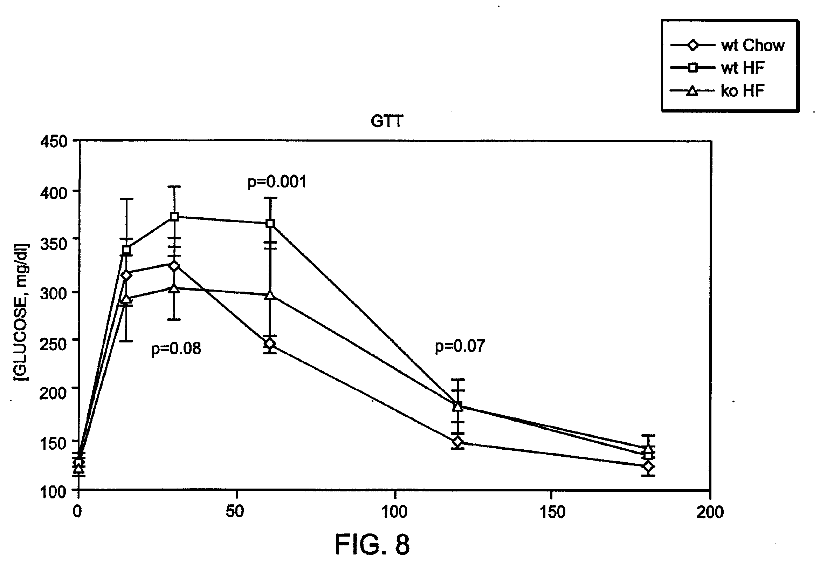

In another embodiment, the phenotype of the FATP1 knockout mouse is manifested as improved glucose clearance in response to a standard glucose tolerance test. As illustrated in FIG. 8, wild-type mice fed a high fat diet manifested a slower clearance rate than wild-type mice fed a standard diet. The clearance rate for FATP1 knockout mice, however, did not significantly differ from wild-type mice fed a standard diet. Again, these experiments indicate potential improvements in insulin utilization in those mice in which the FATP1 transport function was dimimshed.

The acyl-CoA synthetase activity of FATP1 can be measured, for example, by determining the level of acyl-CoA-modified fatty acid accumulation in muscle; consumption of coenzyme A, ATP and fatty acid; and/or production of phosphate, fatty acyl-CoA or AMP. Labeled reagents, *e.g*., fluorescently labeled, enzymatically labeled or radiolabeled, reactants or products can be obtained and detected according to methods known in the art. For example, Consumption of coenzyme A, ATP and fatty acids can be monitored by monitoring the level of radiolabeled coenzyme A, ATP and fatty acids. Alternatively, labeled phosphate, acyl-CoA modified fatty acids or AMP can also be detected and monitored (see Examples 7-9 and 14). Methods for monitoring *in vivo, ex vivo* or *in vitro* levels of these starting materials and products are well known in the art. For example, ³H-labeled palmitic acid or ¹⁴C-labeled lignoceric acid can be used as a substrate for coenzyme A modification and the production of palmitoyl-CoA or lignoceroyl-CoA can be monitored (Coe, N. *et al.,* 1999. *J Biol Chem*, 274;36300-36304).

For example, in one type of cell-based assay, the effect of a modulating compound (*e.g*., an inhibitor) on a FATP1 activity uses a BODIPY-fatty acid (4,4-difluoro -5-methyl-4-bora -3a,4a-diaza-s-indacene-dodecanoic acid). In another Type of cell-based assay, a change of intracellular pH resulting from the uptake of fatty acids can be followed by an indicator fluorophore. The fluorophore can be taken up by the cells in a preincubation step. Fatty acids are added to the cell medium, and after some period of incubation to allow FATP1-mediated uptake of fatty acids, the change in λₘₐₓ of fluorescence can be measured, as an indicator of a change in intracellular pH, as the λₘₐₓ of fluorescence of the fluorophore changes with the pH of its environment, thereby indicating uptake of fatty acids. One such fluorophore is BCECF (2', 7'-bis(2-carboxyethyl)-5(6)- carboxyfluorescein (Rink, T. *et al*., 1982. *J. Cell Biol*., 95:189-196). Thus, for this assay, fatty acid uptake is indicated by a decrease in intracellular pH.

The availability of FATP1 knockout cells and mammals facilitate the genetic dissection of FATP1-mediated signaling pathways and allow for the identification of FATP1 specific inhibitors. For example, an agent that inhibits a function of FATP1 equally in a knockout cell line and its wild-type parental cell line would be recognized as a non-FATP1-specific inhibitor, while an agent that inhibits a FATP1 function in a wild-type cell line and has no effect in the knockout cell line, would be recognized as a FATP1 specific inhibitor.

Other embodiments of the invention provide methods of identifying an agent that inhibits the activity (function) of mammalian FATP1 including, *e.g*., an antagonist, a partial antagonist. An inhibitor of FATP1 includes any agent that inhibits FATP1 gene expression (either a partial or a complete inhibition of expression) or function (either a partial or a complete inhibition of function) of the FATP1 protein. For example, methods described herein for measuring FATP1 activity can be used in screening methods as well as methods known in the art for measuring FATP1 activity. According to the instant invention, the agent can be combined with a cell, a primary tissue, and/or administered to a whole animal. As demonstrated in the following examples, administration can be accomplished in various ways such as the addition to culture media, tissue perfusion, by expressing it from a vector, or by injection.

In one embodiment, an agent can be identified or evaluated based on its ability to inhibit FATP1, thereby reducing insulin resistance. Such a screen can be performed *in vivo* or using cell-based screening methods. For example, an insulin resistant mouse or cells exhibiting insulin resistance can be used for the screening methods described herein. A baseline level, used herein to refer to a level prior to a particular manipulation of a system, for glucose metabolism (*e.g*., uptake or clearance), measured by any of the methods described herein or known in the art, can be determined. Upon addition of a test agent with an amount of insulin normally sufficient to produce an insulin response, glucose metabolism can again be measured. Under insulin resistant conditions, glucose metabolism is eliminated or reduced. If, in the presence of the test agent, glucose metabolism is increased, then insulin resistance has been decreased and the agent is an inhibitor of FATP1

The screening methods described herein can further comprise the use of any suitable control. For example, in one embodiment, the screening method can further comprise optionally inducing insulin resistance in a mammal or providing an insulin resistant non-human mammal, administering to a wild-type mouse an amount of glucose sufficient to monitor an insulin response in the absence of the agent; and administering to a FATP1 knockout mouse an amount of glucose sufficient to stimulate insulin production in the presence of the agent. The rate of glucose uptake of the mice is determined The rate of glucose uptake of the wild-type mouse in the presence of the agent is compared to rate of glucose uptake of the wild-type mouse in the absence of the agent; and the rate of glucose uptake of the FATP1 knockout mouse in the presence of the agent is compared to the rate of glucose uptake of the FATP1 knockout mouse in the absence of the agent. If the rate of glucose uptake of the wild-type mouse in the presence of the agent is increased compared to the rate of glucose uptake by the wild-type mouse in the absence of the agent, and the rate of glucose uptake by the FATP1 knockout mouse in the presence of the agent is similar to the level produced by the knockout mouse in the absence of the agent, then the agent specifically inhibits FATP1. It will be understood by one of skill in the art that the test agent can be administered before, during, or after establishing the insulin resistant state in the mammal.

In the screening methods of the present invention, the rate of glucose taken up the cells, by the wild-type mice or the FATP1 knockout mice can be determined using a variety of methods as described herein or known to those of skill m the art.

Methods of screening agents useful for treating insulin resistance, non-insulin dependent diabetes mellitus and muscle triglyceride accumulation utilizing mammals are also within the scope of the invention. A suitable *in vivo* or *ex vivo* screening method for identifying agents useful for treating insulin resistance comprises optionally inducing insulin resistance in a mammal or providing an insulin resistant non-human mammal, administering to a mammal, tissue or cultured cell line that comprises a wild-type FATP1 gene, *e.g*., a mouse, an amount of glucose sufficient to monitor an insulin response and a test agent. The rate of glucose uptake of the mouse is measured. The rate of glucose uptake of the mouse is compared to the rate of glucose uptake by the same or a similar mouse administered the same amount of glucose in the absence of the test agent. If the rate of glucose uptake of the mouse is increased m the presence of the test agent, then an agent useful in the treatment of insulin resistance has been identified.

Similarly, a suitable *in vivo* screening method for identifying agents useful for treating non-insulin dependent diabetes mellitus comprises optionally inducing insulin resistance in a mammal or providing an insulin resistant non-human mammal, administering to a mammal, *e.g*., a mouse, which comprises a wild-type FATP1 gene, an amount of glucose sufficient to monitor an insulin response and a test agent. The rate of glucose uptake of the mouse is measured. The rate of glucose uptake of the mouse is compared to the rate of glucose uptake by the same or a similar mouse administered the same amount of glucose in the absence of the test agent. If the rate of glucose uptake of the mouse is increased in the presence of the test agent, then an agent useful m the treatment of non-insulin resistant diabetes mellitus has been identified.

*In vivo* and *ex vivo* methods for screening agents useful for modulation of muscle trylyceride accumulation, *e.g*., intracellularly, are also contemplated. Such methods include optionally inducing insulin resistance in a mammal or providing an insulin resistant non-human mammal, administering to a mammal that comprises a wild-type FATP1 gene, *e.g*., a mouse, a test agent. The amount of triglyceride is measured after administration of the test agent and compared to the amount of triglyceride accumulation in cells prior to administration of the test agent. If the amount of triglycerides differ significantly after administration of the test agent, then an agent that modulates cellular triglyceride accumulation has been identified. Such modulation includes both an increase and a decrease in triglyceride. The production or accumulation in cells of various fatty acid metabolites can also be measured in this manner.

The acyl-CoA synthetase activity of FATP1 can be measured, as described above, by *in vivo, ex vivo* and *in vitro* methods. Screening methods based on acyl-CoA synthetase activity can be, for example, the identification of an agent that binds to and inhibits the *in vitro* acyl-CoA synthetase activity of FATP1 as measured, for example, by the cellular production of acyl-CoA-modified fatty acids. Additionally, the cellular accumulation of acyl-CoA-modified fatty acids in cells (*e.g*., adipose, muscle or heart cells), tissues or animals can also be used as an assay for screening for an agent that inhibits FATP1 acyl-CoA synthetase activity.

In one embodiment, the invention is directed to an *in vitro* or an *in vivo* method for identifying an agent useful in treating insulin resistance. As described herein, inhibition of FATP1 leads to a reduction or elimination in insulin resistance. Therefore, an agent that inhibits FATP1 will be useful as an agent that reduces insulin resistance. The method includes contacting a test agent with a mammalian FATP1 (either *in vitro, in vivo,* or *ex vivo, e.g*., in a cultured cell line). The level of acyl-CoA synthetase activity of the mammalian FATP 1 in the presence of the test agent is determined and compared to the level of acyl-CoA synthetase activity of the mammalian FATP1 in the absence of the test agent. When the level of the acyl-CoA syntherase activity is decreased in the presence of the test agent, an agent useful for the treatment of insulin resistance has been identified.

Similarly, in another embodiment, the invention is directed to an *in vitro* or an *in vivo* method for identifying an agent useful in treating non-insulin dependent diabetes mellitus. Since insulin resistance is characteristic of Type 2 diabetes (non-insulin dependent diabetes mellitus), an agent that reduces insulin resistance, *e.g.*, an agent that inhibits FATP1, is useful for treating non-insulin dependent diabetes mellitus. The method includes contacting a test agent with a mammalian FATP1. The level of acyl-CoA synthetase activity of the mammalian FATP1 in the presence of the test agent is determined and compared to the level of acyl-CoA synthetase activity of the mammalian FATP1 in the absence of the test agent. When the level of the acyl-CoA synthetase activity is decreased in the presence of the test agent, an agent useful for the treatment of non-insulin dependent diabetes mellitus has been identified.

*In vitro* or an *in vivo* methods for identifying an agent useful for reducing cellular triglyceride accumulation are also contemplated. The methods include contacting a test agent with a mammalian FATP1. The level of acyl-CoA synthetase activity of the mammalian FATP1 in the presence of the test agent is determined and compared to the level of acyl-CoA synthetase activity of the mammalian FATP1 in the absence of the test agent. When the level of the acyl-CoA synthetase activity differs in the presence of the test agent, an agent useful for the treatment of triglyceride accumulation in muscle has been identified.

In particular embodiments, the levels of acyl-CoA synthetase activity are increased in the presence of the agent. In alternative embodiments, the levels of acyl-CoA synthetase are decreased in the presence of the agent. The altered activity, *i.e.,* modulation of activity, can be measured by the methods described herein.

The present invention also relates to methods of treatment or prevention of conditions (*e.g*., hyperglycemia) or diseases (*e.g*., Type 2 diabetes) affected by FATP1 function. For example the invention provides a method of treating (*e.g*., alleviating the symptoms of) or preventing (*e.g*., in a individual who is predisposed to develop) altered glucose/insulin homeostasis. The term "individual" as used herein is intended to encompass any single member of a species including a human subject. In one embodiment, the invention provides a method of increasing an individual's whole body glucose uptake by administering to the individual an agent that inhibits FAPT1 activity. The increase can result from an increase of insulin-stimulated glucose uptake in muscle cells, in liver cells or both types of cells in combination. In embodiments, the invention also provides a method of decreasing the accumulation of triglycendes in muscle cells.

In another embodiment, the invention provides a method of decreasing blood glucose in an individual comprising administering to the individual an agent that inhibits FATP1 activity. The invention further provides a method of treating diabetes (*e.g*., Type 2 diabetes) in an individual comprising administering to the individual an agent that inhibits FATP1 activity.

The agent for use in the methods of the present invention can be for example, a nucleic acid molecule (*e.g*., DNA, RNA, antisense DNA, antisense RNA), a protein, a peptide, a polypeptide, a glycoprotein, a polysaccharide, an organic molecule, an inorganic molecule, a fusion protein, etc.

The agents (*e.g*., therapeutic agents such as FATP1 inhibitors) can be administered to a host in a variety of ways. Potential routes of administration include intradermal, transdermal (*e.g*., utilizing slow release polymers), intramuscular, intraperitoneal, intravenous, subcutaneous or oral routes. Any convenient route of administration can be used, for example, infusion or bolus injection, or absorption through epithelial or mucocutaneous linings. The agent can be administered in combination with other components such as pharmaceutically acceptable excipients, carriers, vehicles or diluents.

In the treatment methods designed to inhibit the function of FATP1, an "effective amount" of the agent is administered to an individual. As used herein, the term "effective amount", *e.g.,* an amount that inhibits (or reduces) the activity of FATP1, and results in a significant, *e.g*., a statistically significant difference, *e.g.,* increase, decrease, in a cellular function that is normally subject to regulation, *e.g.,* negative regulation by FATP1. For example, an effective amount of a therapeutic agent administered to an individual who is hyperglycemic would comprise an amount sufficient to alter (inhibit) FATP 1 transport of fatty acids that thereby facilitates the effective use of insulin, *i.e*., reduces insulin resistance. The amount of agent required to inhibit FATP1 activity will vary depending on a variety of factors including the size, age, body weight, general health, sex and diet of the host as well as the time of administration, and the duration or stage of the particular condition or disease that is being treated. Effective dose ranges can be extrapolated from dose-response curves derived *in vitro* or an *in vivo* test system that utilizes, for example, the non-human FATP1 knockout mice described herein.

All patents, patent applications and references referred to herein are incorporated by reference in their entireties.
- The following examples are offered for the purpose of illustrating the present invention and are not to be construed to limit the scope of this invention. The experimental procedures were utilized as described in the relevant experiments as described in the Examples that follow.

### EXPERIMENTAL PROCEDURES

### Methods

Animals and surgery: Male FATP1 knockout (FATP1 KO) and wild-type littermates (control) weighing ∼30 g (∼16 weeks of age) were studied to examine the effect of chronic high fat feeding and acute lipid infusion on insulin action and signaling. High fat diet (Harlan Teklad, Madison, WI), consisting of 55 % fat, was fed *ad* *libitum* for 3 weeks in the high fat diet group. At least 4 days before hypennsulinemic-euglycemic clamp experiments, mice were anesthetized with an intraperitoneal injection of ketamine (100 mg/kg body weight) and xylazine (10 mg/kg body weight), and an indwelling catheter was inserted in the left internal jugular vein (Kim, J., *et al*., *2000 J Biol Chem*., 275, 8456-8460) In order to conduct experiments in conscious mice causing minimal stress, a tail restraint method was employed.

Hyperinsulinemic-euglycemic clamp to assess *in vivo* insulin action in awake mice: After an overnight fast, saline or lipid (5 ml/kg/hr, Liposyn II, triglyceride emulsion, 20% wt/vol, Abbott, North Chicago, IL) and heparin (6 U/hr) were infused for 5 hours to raise plasma fatty acids concentrations. A blood sample (60 µl) was collected at the end of 5-hour lipid infusion period for measurement of basal concentrations of glucose, insulin, and fatty acids. HPLC-purified [3-³H]glucose (0.05 µCi/min; NEN) was infused during The basal period to estimate the rate of basal glucose turnover. A 120 minute hyperinsulinemic-euglycemic clamp was conducted with a primed-continuous infusion of human insulin (Humulin; Eli Lilly) at a rate of 15 pmol/kg/min to raise plasma insulin within a physiological range (∼700 pM). Blood samples (20 µl) were collected at 20 minutes intervals for the immediate measurement of plasma glucose concentration, and 20% glucose was infused at variable rates to maintain plasma glucose at basal concentrations. Insulin-stimulated whole body glucose flux were estimated using a primed-continuous infusion of [3-³H]glucose (10 µCi bolus, 0.1 µCi/min) throughout the clamps. All infusions were done using microdialysis pumps (CMA/Microdialysis).

To estimate insulin-stimulated glucose uptake in individual tissues, 2-deoxy-D-[1-¹⁴C]glucose(2-[¹⁴C]DG; NEN) was administered as a bolus (10 µCi) at 75 minutes after the start of clamps. Blood samples (20 µl) were taken at 80, 85, 90, 100, 110, and 120 minutes after the start of clamps for the determination of plasma [³H]glucose, ³H₂O, and 2-[¹⁴C]DG concentrations, Additional blood samples (40 µl) were collected at the end of clamps for measurement of plasma insulin and fatty acids concentrations. At the end of clamps, mice were anesthetized with sodium pentobarbital injection. Within 5 minutes, three muscles (gastrocnemius, tibialis anterior, and quadriceps) from both hindlimbs, epididymal white adipose tissue, intrascapular brown adipose tissue, and liver were taken. Each tissue, once exposed, was dissected out within 2 seconds, frozen immediately using liquid N2-cooled aluminum blocks, and stored at -70°C for later analysis. Plasma glucose concentration during clamps was analyzed using 10 µl plasma by a glucose oxidase method on a Beckman glucose analyzer 2 (Beckman).

Plasma insulin concentration was measured by radioimmunoassay (R1A) using kits from Linco Research. Plasma fatty acids concentration was determined using an acyl-CoA oxidase-based colorimetric kit (Wako Chemicals USA). For the determination of plasma [3-³H]glucose and 2-[¹⁴C]DG concentrations, plasma was deprotemized with ZnSO₄ and Ba(OH)₂, dried to remove ³H₂O, resuspended in water, and counted in scintillation fluid (Ultima Gold; Packard) on dual channels for separation of ³H and ¹⁴C.

The plasma concentration of ³H₂O was determined by the difference between ³H counts without and with drying. The radioactivity of ³H in tissue glycogen was determined by digesting tissue samples in KOH and precipitating glycogen with ethanol as previously described (Kam, J. *et al*., 1996. *Diabetes*, 45:446-453). For the determination of tissue 2-[¹⁴C]DG-6-phosphate (2-DG-6-P) content, tissue samples were homogenized, and the supernatants were subjected to an ion-exchange column to separate 2-DG-6-P from 2-DG. Rates of basal and insulin-stimulated whole body glucose turnover were determined as the ratio of the [³H]glucose infusion rate (disintegrations per minute; dpm/min) to the specific activity of plasma glucose (dpm/µmol) at the end of basal period and during the final 30 minutes of clamps, respectively. Hepatic glucose production during the hyperinsulinemic-euglycemic clamps was determined by subtracting the glucose infusion rate from the whole body glucose uptake. Whole body glycolysis was calculated from the rate of increase in plasma ³H₂O concentration, determined by linear regression of the measurements at 80, 90, 100, 110, and 120 minutes.

Whole body glycogen and lipid synthesis was estimated by subtracting whole body glycolysis from whole body glucose uptake, assuming that glycolysis and glycogen/lipid synthesis account for the majority of insulin-stimulated glucose uptake (Rossetti, L. and Giaccari, A., 1990. *J. Clin. Invest,* 85:1785-1792). Glucose uptake in individual tissues was calculated from plasma 2-[¹⁴C]DG profile, which was fitted with a double exponential curve using MLAB (Civilized Software) and tissue 2-DG-6-P content. Skeletal muscle glycogen synthesis was calculated from ³H incorporation to muscle glycogen. Skeletal muscle glycolysis was then estimated as the difference between muscle glucose transport and muscle glycogen synthesis.

### Example 1. Generation of a FATP1 targeting construct.

Genomic DNA containing the mouse FATP1 locus was obtained by screening a 129/Sv genomic bacterial artificial chromosome library (Research Genetics) with a fragment containing the 5' end of the mouse FATP1 coding sequence. Positive clones were digested with *Eco*RI and *Sac*I. FATP1-containing fragments were identified by Southern blotting and subcloned into a standard shuttle vector. To obtain arms for a targeting construct, PCR primers were designed that allowed amplification of 2.3 kb of genomic DNA just upstream of the initiation codon (5' arm) and 2 kb of genomic DNA just downstream of the first coding exon (3' arm). The PCR primers were designed to introduce an *Eco*RI site at the 3' end of the 5' arm and a *Sac*I site at the 5' end of the 3' arm. The amplified arms were subcloned into the targeting vector pGEMneo-lacZ. pGEMneo-lacZ is a:pGEM-based vector (Promega) containing a PGK-neo expression cassette (consisting of the neomycin gene under the transcriptional control of the mouse phosphoglycerate kinase (PGK-1) promoter and the PGK-1 poly(A) addition site), as well as a promoter less lacZ gene containing a nuclear localization sequence (Mercer, E. *et al,* 1991. *Neuron,* 7:703-716). The lacZ coding sequence is located immediately downstream of the 5' arm, thus putting lacZ under the transcriptional control of the FATP1 promoter.

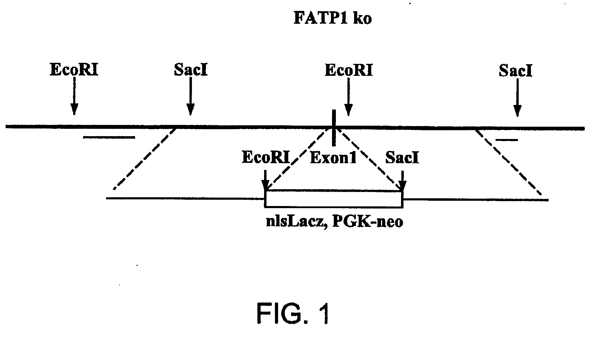

To confirm recombination events by Southern Blot, probes were generated from genomic DNA immediately adjacent to the arms present in the targeting construct. To generate the 5' probe, a 1.2 kb fragment of genomic DNA starting immediately upstream of the 5' arm was amplified by PCR and subcloned into a shuttle vector. To generate a 3' probe, a 400 nucleotide fragment of genomic DNA starting 90 nucleotides downstream of the 3' arm was amplified by PCR and subcloned into a shuttle vector. The design of targeting vector, probes and the predicted structure of the FATP1 genomic locus for a correct recombination event is summarized in FIG. 1. The correct recombination event results in deletion of the first 56 ammo acids of the FATP1 protein and replaces the first coding exon of the FATP1 gene with The lacZ gene followed by the PGK-neo cassette.

### Example 2: Generation of targeted ES cells.

The 129SvEv ES cell line was cultured on SNL76/7 mitotically inactive feeder cells as described by Robertson (in *Teratocarcinomas and Embryonic Srem Cells: A practical approach*, ed. Robertson, E. J., IRL, Oxford, 1987, pp71-112). Electroporation of the cells was then performed (Huszar, D. *et al*., 1997. *Cell,* 88:131-141). Briefly, cells were trypsinized and resuspended at a concentration of 1.0 x 10⁷ ml in PBS (Ca and Mg-free, Gibco). A 0.7 ml aliquot (7 x 10⁶ cells) was mixed with 20 µg of the linearized targeting vector and pulsed at 250 V, 500 µF (Bio-Rad Gene Pulser). The cells were then diluted in culture medium, plated at 1.2 x 10⁶ per 100 mm plate containing feeder cells and placed under selection 24 hours later in G418 sulfate (300 µg/ml solution; Gibco). G418-resistant clones were picked, dissociated with trypsin, and divided into one well each of two 96-well plates. Upon confluence, ES cells were frozen in one of the 96-well plates (Ramirez-Solis, R. *et al*., 1993. *Methods Enzymol*., 225:855-878). DNA was prepared from the other plate for screening by Southern blor hybridization. Positive clones were extended and analyzed by Southern Blot hybridization using 5' and 3' flanking probes to confirm recombination.

### Example 3: Generation of FATP1-deficient mice.

ES cell clones that had undergone homologous recombination were injected into blastocysts that were then transferred to pseudopregnant female mice to generate chimeric offspring. Male chimeras were mated with C57B1/6J females to obtain germline transmission of the disrupted FATP1 gene. The resulting heterozygotes were interbred to generate mice homozygous or heterozygous for the FATP1 mutation, along with wild-type litter mates

### Example 4: Rate of glucose disappearance (Insulin-stimulated whole body glucose uptake).

Three groups of sixteen week old male wild-type mice were tested. Group 1 received no treatment. Group 2 received a lipid infusion for five hours. Group 3 received a high fat diet for three weeks. Three groups of sixteen week old male FATP1 knockout mice were also tested. Group 1 received no treatment. Group 2 received a lipid infusion for five hours. Group 3 received a high fat diet for three weeks. All six groups of mice were treated with a euglycemic hyperinsulinemic clamp. As shown FIG. 2, the knockout mice in Groups 2 and 3 exhibited a statistically significantly increased rate of glucose disappearance when compared to the corresponding group of wild-type mice.

### Example 5: Gastrocnemius glucose transport.

Three groups of sixteen week old male wild-type mice were tested. Group 1 received no treatment. Group 2 received a lipid infusion for five hours. Group 3 received a high fat diet for three weeks. Three groups of sixteen week old male knockout mice were rested. Group 1 received no treatment. Group 2 received a lipid infusion for five hours. Group 3 received a high fat diet for three weeks. All six groups of mice were treated with a euglycemic hyperinsulinemic clamp. As shown in FIG. 3, the gastrocnemius muscle of the knockout mice in Groups 2 and 3 exhibited a significantly increased rate of glucose uptake when compared to the corresponding group of wild-type mice.

### Example 6: White adipose tissue glucose transport

Three groups of sixteen week old male wild-type mice were tested. Group 1 received no treatment. Group 2 received a lipid infusion five hours Group 3 received a high fat diet for three weeks. Three groups of sixteen week old male knockout mice were tested. Group 1 received no treatment. Group 2 received a lipid infusion for five hours. Group 3 received a high fat diet for three weeks. All six groups of mice were treated with a euglycemic hyperinsulinemic clamp. As shown in FIG. 4, the knockout mice in Group 2 exhibited an increased rate of glucose disappearance when compared to the wild-type mice.

### Example 7: Triglyceride levels in muscle tissue

Three groups of sixteen week old male wild-type mice were tested. Group 1 received no treatment. Group 2 received lipid infusion for five hours. Group 3 received a high fat diet for three weeks. Three groups of sixteen week old male knockout mice were tested. Group 1 received no treatment. Group 2 received a lipid infusion for five hours. Group 3 received a high fat diet for three weeks. All six groups of mice were treated with a euglycemic hyperinsulinemic clamp. Triglyceride levels were measured in quadricep muscle tissue. As shown in FIG. 5, the wild-type mice showed a ten-fold increase in muscle triglyceride when fed a high-fat diet, while FATP1 knockout mice were completely protected from triglyceride accumulation in the muscle when fed the same diet.

### Example 8: Liver triglyceride levels.

Three groups of sixteen week old male wild-type mice were tested. Group 1 received no treatment. Group 2 received a lipid infusion for five hours. Group 3 received a high fat diet for three weeks. Three groups of sixteen week old male knockout mice were rested. Group 1 received no treatment Group 2 received a lipid infusion for five hours. Group 3 received a high fat diet for three weeks. All six groups of mice were treated with a euglycemic hypermsulmemic clamp. Trigylceride levels were measured in liver tissue. As shown in FIG. 6, the wild-type mice showed a two-fold increase in liver triglyceride when fed a high fat diet, while FATP1 knockout mice were partially protected from triglyceride accumulation in the liver when fed the same diet.

### Example 9. Screening for an agent that modulates triglyceride uptake

Human embryonic kidney 293 cells stably transfected with pIRES-FATP vector were cultured and passaged in DMEM base medium. 8 x10⁴ cells per well were plated in a 96-well microtiter plate, and washed with washing buffer (20 mM HEPES, 4.2 mM NaHCO₃ in IX Hanks basic salt solution). The inhibitor in 1 mM taurocholate in washing buffer is Then added to the cells at 37°C. The uptake assay is initiated by addition of 5 mM final BODIPY-fatty acid (4,4-difluoro -5-methyl -4-bora -3a,4a-diaza -s-indacene-dodecanoic acid). The uptake assay is run for 15 to 60 minutes, and stopped by washing with 0.1% bovine serum albumin in washing buffer. The amount of BODIPY-fatty acid taken up by the cells is then determined by measuring the fluorescence of the cells after washing (excitation = 450 nm, emission = 530 nm). The inhibition of uptake as a function of concentration of inhibitor was graphed and fit to the equation (Y = mix + (mix - mm)/(1+10^((log IC₅₀-X)*HillSlope)) to calculate IC₅₀ value for the inhibitor.

### Example 10: Inhibition of acyl-CoA activity reduces insulin resistance.

Insulin action on glucose uptake and metabolism was examined during a 2-hour hyperinsulinemic-euglycemic clamp (see above) m awake, age-matched, male wild-type and FATP1 knockout mice following a 5-hour lipid infusion or a 3-week feeding f high fat diet (55% fat by calories; see above).

Overnight fasted plasma glucose concentrations did not differ between wild-type and FATP1 knockout mice fed regular mouse chow diet but showed a tendency to increase in the wild-type mice following the high fat diet. During the clamps (see "Experimental Procedures"), plasma insulin concentration was raised to ∼450 pM, while the plasma glucose concentration was maintained at ∼7 mM by a variable infusion of glucose in all groups. Plasma fatty acid concentrations were elevated by ∼2-3 fold following a 5-hour lipid infusion or 3 weeks of high fat feeding in the wild-type and FATP1 knockout mice (Fig. 9A). The basal hepatic glucose production (HGP) and insulin's ability to suppress basal HGP were not different in the wild-type and FATP1 knockout mice (105±23 and 87±15 µmol/kg/min for basal HGP, respectively, and 16±14 and 10±9 µmol/kg/min for HGP during insulin-stimulated state, respectively).

The glucose infusion rate required to maintain euglycemia increased rapidly in the wild-type and FATP1 knockout mice fed regular mouse chow diet (control group) and reached a steady state within 90 minutes. In contrast, there was a markedly blunted insulin response during the hyperinsulinemic-euglycemic clamp studies in the wild-type mice after lipid infusion or high fat feeding, as reflected by a much lower steady state glucose infusion rate (Fig. 9B). In contrast, lipid infusion or high fat feeding did not affect steady state glucose infusion rate in the FATP1 knockout mice (FIG. 9B).

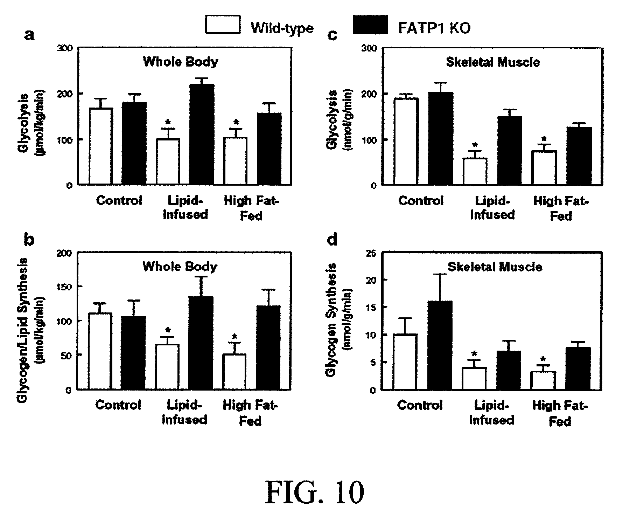

Insulin-stimulated whole body glucose turnover was decreased by ∼40% in the wild-type mice following lipid infusion or high fat feeding whereas it remained normal in the FATP1 knockout mice (FIG. 9C). The rate of insulin-stimulated glucose uptake in skeletal muscle *in vivo* was estimated using 2-deoxy-D-[1-¹⁴C]glucose injection during hyperinsulinemic-euglycemic clamps in awake mice. Since 2-deoxyglucose is a glucose analog that is phosphorylated but not metabolized, tissue-specific rates of insulin-stimulated glucose uptake can be estimated by determining the tissue content of 2-deoxyglucose-6-phosphate. Lipid infusion or high fat feeding decreased insulin-stimulated glucose uptake in skeletal muscle (gastrocnemius) of the wild-type mice by ∼70%. In contrast, FATP1 knockout mice were protected from fat-induced decreases in insulin-stimulated glucose uptake in skeletal muscle (FIG. 9D). Insulin-stimulated whole body glycolysis and glycogen/lipid synthesis were decreased by ∼40% and ∼50% in the wild-type mice following lipid infusion or high fat feeding, respectively (FIGS. 10A and 10B). In contrast, lipid infusion or high fat feeding did not affect insulin-stimulated whole body glucose metabolism in the FATP1 knockout mice. Furthermore, insulin-stimulated glycolysis and glycogen synthesis in skeletal muscle (gastrocnemius) were decreased by ∼70% and ∼80% in the wild-type mice following lipid infusion or high fat feeding, respectively (FIGS. 10C and 10D). In contrast, FATP1 knockout mice were protected from fat-induced decreases in insulin-stimulated muscle glucose metabolism.

These results indicate an important role of FATP1 and fatty acids uptake in skeletal muscle in the development of fat-induced insulin resistance.

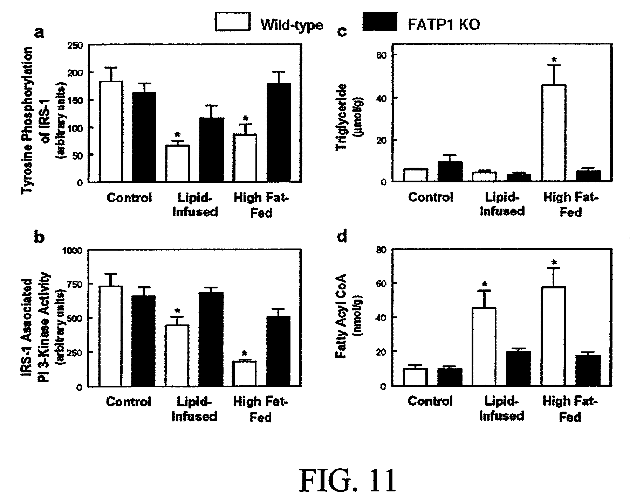

Recent studies in IRS-1 gene disrupted mice have suggested that IRS-1 is important in insulin activation of glucose transport and glycogen synthase activity in skeletal muscle (Previs, S. *et al*., 2000. *J Biol. Chem.,* 275:38990-38994). Insulin-stimulated IRS-1 tyrosine phosphorylation in skeletal muscle (gastrocnemius) was decreased by 60% and 50% in the wild-type mice following lipid infusion and high fat feeding, respectively (FIG, 11A). Similarly, insulin-stimulated IRS-1 associated PI 3-kinase activity in skeletal muscle (gastrocnemius) was decreased by 40% and 75% in the wild-type mice following lipid infusion and high fat feeding, respectively (FIG. 11B). In contrast, FATP1 knockout mice were protected from fat-induced defects in insulin-stimulated IRS-1 tyrosine phosphorylation and IRS-1 associated P1 3-kinase activity in skeletal muscle (FIGS. 11A and 11B).

Defects in skeletal muscle insulin action following lipid infusion or high fat diet may be due to increases in intramuscular fat and fatty acid-derived metabolites. Previous studies have shown a strong inverse relationship between intramuscular fat content and insulin sensitivity in humans and animals (K. Krssak *et al,* 1999. *Diaberologia,* 42:113-116; Perseghin, G. *et al*., 1999. *Diabetes,* 48:1600-1606; Elhs, B. *et al*., 2000. *Am. J. Physiol. Endocrinol. Metab.,* 279, E554-E560; Montell, E. *et al*., 2001. *Am. J. Physiol. Endocrinol. Metab*., 280:E229-E237; Kim, J. *et al*., 2001. *Proc. Natl. Acad. Sci. USA,* 98:7522-7527). In order to examine whether the fat-induced changes in skeletal muscle insulin action were associated with changes in intramuscular fat contents, triglyceride and fatty acyl-CoA concentrations were measured in skeletal muscle using liquid chromatography tandem spectrometry (LC/MS/MS). Intramuscular (quadriceps) triglyceride concentration was increased in the wild-type mice following high fat feeding but remained normal in the FATP1 knockout mice (FIG. 11C). Additionally, intramuscular fatty acyl-CoA concentrations were elevated in the wild-type mice following lipid infusion or high fat feeding whereas FATP1 knockout mice were protected from fat-induced increases in intramuscular fatty acid-denved metabolites (FIG. 11D).

### Example 11: Determination of changes in body weight.

Two groups of twenty-six week old male wild-type mice were tested for changes in body weight. Group 1 was fed a standard diet. for twelve weeks. Group 2 received a high fat diet (D12330, Research Diets, New Brunswick, NJ) for twelve weeks. Two groups of twenty-six week old male heterozygous mice were tested for changes in body weight. Group 1 was fed a standard diet for twelve weeks. Group 2 received a high fat diet (D12330, Research Diets, New Brunswick, NJ) for twelve weeks. Two groups of twenty-six week old male FATP1 knockout mice were tested for changes in body weight. Group 1 was fed a standard diet for twelve weeks. Group 2 received a high fat diet (D12330, Research Diets, New Brunswick, NJ) for twelve weeks. As illustrated in FIG. 7A, all the groups receiving high fat diets exhibited increased body weight.

### Example 12: Determination of changes to Fat/Lean tissue ratios.

A DEXA scan was performed on twenty-six week old male wild-type mice fed a standard diet. DEXA scans were also performed on twenty-six week old male wild-type mice and twenty-six week old male knockout mice receiving a high fat (D 12330, Research Diets, New Brunswick, NH) during the past twelve weeks. As illustrated in FIG. 7B, the mice receiving the high fat diets exhibited increased body fat.

### Example 13: Glucose tolerance tests.

Glucose tolerance tests were performed on twenty-six week old male wild-type mice fed a standard diet. Glucose tolerance tests were also performed on twenty-six week old male wild-type mice and sixteen week old male knockout mice receiving a high fat diet for twelve weeks. Mice were fasted ovemight, injected i.p. with 2.0 mg glucose per gram of body weight, and blood glucose concentration was determined at the indicated times after injection using a standard glucometer (Bayer, Elkhard, IN) according to the manufacturer's instructions. As illustrated in FIG. 8, wild-type mice fed high fat diets cleared the glucose sigruficantly less rapidly than - those fed a standard diet. In contrast, knockout mice fed a high-fat diet exhibited clearance rates that were not significantly different from wild-type mice on a standard diet.

### Example 14; Acyl-CoA synthetase activity by FATP1

*Expression and purification of hsFATP1*. The human FATP1 coding sequence containing a C-terminal 6x-HIS tag is inserted into the pFastBAC vector and Baculovirus expressing hsFATP1-His using established methods. Sf9 insect cells are infected with hsFATP1-His containing baculovirus, harvested 48 hours later and frozen in liquid nitrogen. Cells are thawed on ice in lysis buffer (50 mM Tris, pH 8.0, 20 mM imidazole, 1 M NaCl, 0.1% NP-40, 10% glycerol, 5 mM 2-Mercapto-ethanol containing a standard cocktail of protease inhibitors) and lysed by sonication. The lysate is spun at 17,700 g for 30 minutes at 4°C and the supernatant is applied to a Ni-NTA superflow resin (Qiagen) equilibrated in lysis buffer, 500 ml resin is used per 0.5 L of cells. The resin is incubated at 4°C for 1 hour with gentle shaking, centrifuged at 400 g for 15 minutes at 4°C, and the resin is then transferred into a 10 ml disposable column. The column is washed with 40 column volumes of equilibration buffer, and bound material is eluted by adding 2 column volumes of elution buffer (50 mM Tris, pH 8.0, 0.3 M inudazole, 1 M NaCl, 0.1% NP-40, 10% glycerol, 5 mM 2-Mercapto-ethanol).

*Assay for Acyl-CoA synthetase activity*. Acyl-CoA synthetase activity assays are performed as described (Choi, J. and Martin, C., 1999 *J. Biol. Chem.,* 274:4671-4683). Briefly, 10 µg of crude supernatant, or 3 µg of column-purified material is incubated with 10 µM radiolabeled palmitate or lignocerate solubilized in α-cyclodextrin for 30-60 minutes at 37°C. The material is exiracted as described by Choi and Martin, and the aqueous phase is counted in a scintillation counter. Activity is expressed as pmol/min/mg protein.

While this invention has been particularly shown and described with references to preferred embodiments thereof, it will be understood by those skilled in the art that various changes in form and details may be made therein without departing from the scope of the invention encompassed by the appended claims.

## Claims

1. A method for identifying a candidate agent useful for the treatment of an FATP1-mediated metabolic disorder comprising:
a) contacting a test agent with a composition comprising mammalian FATP1;
b) measuring the activity of FATP1 the presence of the test agent;
c) comparing the level of FATP1 activity in the presence of the test agent to the level of FATP1 activity in the absence of the test agent; and
d) identifying the agent as a candidate agent useful for the treatment of an FATP1 mediated metabolic disorder where the level of the FATP1 activity is altered in the presence of the test agent;
wherein the FATP1 activity measured is selected from the group consisting of acyl-CoA synthetase enzyme activity, fatty acid transport, and glucose uptake; and
wherein the FATP1-mediated disorder is selected from the group consisting of insulin resistance, non-insulin dependent diabetes mellitus, and cellular triglyceride accumulation.

2. The method of Claim 1 wherein the composition comprising mammalian FATP1 is selected from the group consisting of: purified FATP1 polypeptide, membrane preparations comprising FATP1, and cells comprising FATP1.

3. The method of Claim 1 wherein the activity of FATP1 is measured using an assay selected from the group consisting of: an *in vitro* acyl-CoA synthetase enzyme assay, a cell-based fatty acid transport assay, a tissue-based fatty acid transport assay, or an *in vivo* assay.

4. The method of Claim 3 wherein the acyl-CoA synthetase activity of FATP1 is determined by measuring the production of a product selected from the group consisting of phosphate, fatty acyl-CoA and AMP.

5. The method of Claim 3 wherein the acyl-CoA synthetase activity of FATP1 is determined by measuring consumption of a substrate selected from the group consisting of coenzyme A, ATP, and fatty acid.

6. The method of claim 1 further comprising:
e) administering the identified candidate agent to a mammal having insulin resistance;
f) determining a level of FATP1-mediated biological activity of the mammal;
g) comparing the biological activity of the mammal administered the agent with the biological activity of a mammal not admmistered agent, and
h) identifying the agent as an agent useful for the treatment of an FATP1-mediated metabolic disorders when the biological activity of the mammal administered agent is altered from the biological activity of the mammal not administered agent;
wherein the biological activity measured is selected from the group consisting of glucose uptake, glucose clearance, triglyceride accumulation, fatty acid transport, and FATP1 mediated acyl-CoA synthetase activity; and
wherein the FATP1-mediated disorder is selected from the group consisting of insulin resistance, non-insulin dependent diabetes melhtus, and muscle triglyceride accumulation.

7. The method of claim 6 wherein the test agent is administered prior to, after, or substantially simultaneously with inducing insulin resistance.

8. The method of claim 6 wherein insulin resistance is induced by a high fat diet or by lipid infusion.

9. The method of claim 6 wherein insulin is administered to the mammal prior to, after, or substantially simultaneously with the agent..

10. A method for identifying an agent that inhibits an FATP1 activity comprising:
a) optionally inducing insulin resistance in a non-human mammal or providing an insulin resistant non-human mammal;
b) administering an agent to the mammal;
c) determining a biological activity level of the mammal; and
d) comparing the biological activity of the mammal administered the agent with the biological activity of a mammal not administered agent,
wherein the biological activity measured is selected from the group consisting of glucose uptake, glucose clearance, muscle triglycende accumulation, fatty acid transport, and FATP1 mediated acyl-CoA synthetase activity; and
whereby if the biological activity of the mammal that had been administered the agent is altered from the biological activity of the mammal that not been administered the agent, then the agent inhibits FATP1.

11. The method of Claim 10 wherein the test agent is administered prior to, after, or substantially simultaneously with inducing insulin resistance.

12. The method of Claim10 wherein insulin resistance is induced by a high fat diet or by lipid infusion.

13. The method of Claim 10 wherein insulin is administered to the mammal prior to, after, or substantially simultaneously with the agent.

14. A method for identifying an agem that inhibits an FATP1 activity comprising:
a) optionally inducing insulin resistance in a non-human mammal comprising a wild-type FATP1 gene and a non-human mammal comprising a disrupted FATP1 gene, or providing an insulin-resistant non-human mammal comprising a wild-type FATP1 gene and a non-human mammal comprising a disrupted FATP1 gene;
b. administering an agent to the mammal comprising a wild-type FATP 1 gene;
c. administering an agent to the mammal comprising a disrupted FATP1 gene;
d. determining a biological activity level of the mammals; and
e. comparing the biological activity of the mammal whose genome comprises a wild-type FATP1 gene and the mammal whose genome comprises a disrupted FATP1 gene,
wherein the biological activity measured is selected from the group consisting of glucose uptake, glucose clearance, muscle triglyceride accumulation, fatty acid transport, and FATP1 mediated acyl-CoA synthetase activity; and
whereby if the biological activity of the mammal whose genome comprises a wild-type FATP1 gene is similar to the biological activity of the mammal whose genome comprises a disrupted FATP1 gene, then the agent inhibits FATP1.

15. The method of Claim 14 wherein the mammal in which the genome comprises a disruption of the FATP1 gene is a homozygous disruption or a heterozygous disruption.

16. The method of Claim 14 wherein the test agent is administered prior to, after, or substantially simultaneously with inducing insulin resistance.

17. The method of Claim 14 wherein insulin resistance is induced by a high fat diet or by lipid infusion.

18. The method of Claim 14 wherein insulin is administered to the mammal prior to, after, or substantially simultaneously with the agent.

19. A method of modulating an FATP1 mediated disorder in an individual comprising administering to the individual an effective amount of an agent that inhibits FATP1 activity.

20. The method of claim 19 wherein modulation an FATP1-mediated disorder comprises any one of reducing insulin resistance; increasing insulin stimulated whole body glucose uptake; decreasing blood glucose; reducing muscle triglyceride accumulation; or treating non-insulin dependent diabetes mellitus in an individual.

21. A non-human mammal with a genome comprising a disruption of a FATP1 gene such that the mammal lacks or has reduced levels of functional FATP1 protein, and wherein the mammal exhibits an altered insulin/glucose homeostasis and responsiveness to glucose stimulation compared to a wild-type or non-transgenic mammal.

22. A method of producing the non-human mammal of Claim 21 comprising:
a) introducing a targeting vector that disrupts the FATP1 gene in an embryonic stem cell, thereby producing a transgenic embryonic stem cell that comprises a disrupted FATP1 gene;
b) selecting a transgenic embryonic stem cell that comprises the disrupted FATP1 gene;
c) introducing the selected transgeme embryonic stem cell into a blastocyst, thereby forming a chimeric blastocyst; and
d) introducing the chimeric blastocyst into the uterus of a pseudopregnant mammal; whereby the pseudopregnant mammal gives birth to a transgenic mammal with a genome comprising a disruption of a FATP1 gene such that the mammal lacks or has reduced levels of functional FATP1 protein, and wherein the mammal exhibits an altered insulin/glucose homeostasis and responsiveness to glucose stimulation compared to a wild-type or non-transgenic mammal.

23. A method for identifying an agent that inhibits an FATP1 activity, using data on a biological activity level obtained from a mammal to which the agent has been administered and from a mammal to which the agent has not been administered comprising:
a) comparing the biological activity data of the mammal administered the agent with the biological activity data of the mammal not administered the agent
wherein the biological activity measured is selected from the group consisting of glucose uptake, glucose clearance, muscle triglyceride accumulation, fatty acid transport, and FATP1 mediated acyl-CoA synthetase activity; and
whereby if the biological activity of the mammal that had been administered the agent is altered from the biological activity of the mammal that had not been administered the agent, then the agent inhibits FATP1.

24. A method for identifying an agent that inhibits an FATP1 activity, using data on a biological activity level obtained from a mammal comprising a wild-type FATP1 gene to which the agent has been administered and from a mammal comprising disrupted FATP1 gene to which the agent has been administered comprising:
a) comparing the biological activity data of the mammal whose genome comprises a wild-type FATP1 gene with the mammal whose genome comprises a disrupted FATP1 gene,
wherein the biological activity measured is selected from the group consisting of glucose uptake, glucose clearance, muscle triglyceride accumulation, fatty acid transport, and FATP1 mediated acyl-CoA synthetase activity; and
whereby if the biological activity of the mammal whose genome comprises a wild-type FATP1 gene is similar to the biological activity of the mammal whose genome comprises a disrupted FATP1 gene, then the agent inhibits FATP1.

25. A method for identifying an agent useful for the treatment of an FATP1-mediated metabolic disorder using data on a level of FATP1-mediated biological activity obtained from a mammal to which a candidate agent identified according to any one of claims 1 to 5 has been administered and from a mammal not administered the agent comprising:
a) comparing the biological activity data of the mammal administered the agent with the biological activity data of the mammal not administered the agent; and
b) identifying the agent as an agent useful for the treatment of a FATP1-mediated metabolic disorder when the biological activity of the mammal administered the agent is altered from the biological activity of the mammal not administered the agent;
wherein the biological activity measured is selected from the group consisting of glucose uptake, glucose clearance, muscle triglyceride accumulation, fatty acid transport, and FATP1 mediated acyl-CoA synthetase activity; and
wherein the FATP1-mediated disorder is selected from the group consisting of insulin resistance; non-insulin dependent diabetes mellitus, and muscle triglyceride accumulation.

26. Use of an agent that inhibits FATP1 activity for the manufacture of a medicament for modulating a FATP1 mediated disorder.

27. A method of producing a chimeric blastocyst for producing the non-human mammal of claim 21 comprising:
a) introducing a targeting vector that disrupts the FATP1 gene in an embryonic stem cell, thereby producing a transgenic embryonic stem cell that comprises a disrupted FATP1 gene;
b) selecting a transgenic embryonic stem cell that comprises the disrupted FATP1 gene; and
c) introducing the selected transgenic embryonic stem cell into a blastocyst, thereby forming a chimeric blastocyst.
